(19) Europäisches Patentamt
European Patent Office
Office européen des brevets

(11) EP 3 287 126 A1

(12) EUROPEAN PATENT APPLICATION

(43) Date of publication:
**28.02.2018 Bulletin 2018/09**

(21) Application number: **17179348.2**

(22) Date of filing: **01.03.2010**

(51) Int Cl.:
***A61K 31/045*** $^{(2006.01)}$ ***A61K 31/4375*** $^{(2006.01)}$
***A61K 45/06*** $^{(2006.01)}$ ***A61P 35/00*** $^{(2006.01)}$

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.02.2009 US 156449 P**
**16.04.2009 US 170013 P**

(62) Document number(s) of the earlier application(s) in accordance with Art. 76 EPC:
**10707173.0 / 2 400 959**

(27) Previously filed application:
**01.03.2010 PCT/US2010/025737**

(71) Applicant: **Sunesis Pharmaceuticals, Inc.**
**South San Francisco, CA 94080 (US)**

(72) Inventors:
- **HAWTIN, Rachael, E.**
**San Carlos CA 94070 (US)**
- **FOX, Judith, A.**
**San Francisco CA 94115 (US)**

(74) Representative: **Jones Day**
**Rechtsanwälte, Attorneys-at-Law, Patentanwälte**
**Prinzregentenstrasse 11**
**80538 München (DE)**

Remarks:
•This application was filed on 03-07-2017 as a divisional application to the application mentioned under INID code 62.
•Claims filed after the date of filing of the application (Rule 68(4) EPC).

# (54) METHODS OF USING SNS-595 FOR TREATMENT OF CANCER SUBJECTS WITH REDUCED BRCA2 ACTIVITY

(57) Methods of using SNS-595 for treatment of a subject having cancer with BRCA2 mutation are described. In certain embodiments, the methods comprise administering a therapeutically effective amount of SNS-595 to a subject in need thereof.

FIGURE 1

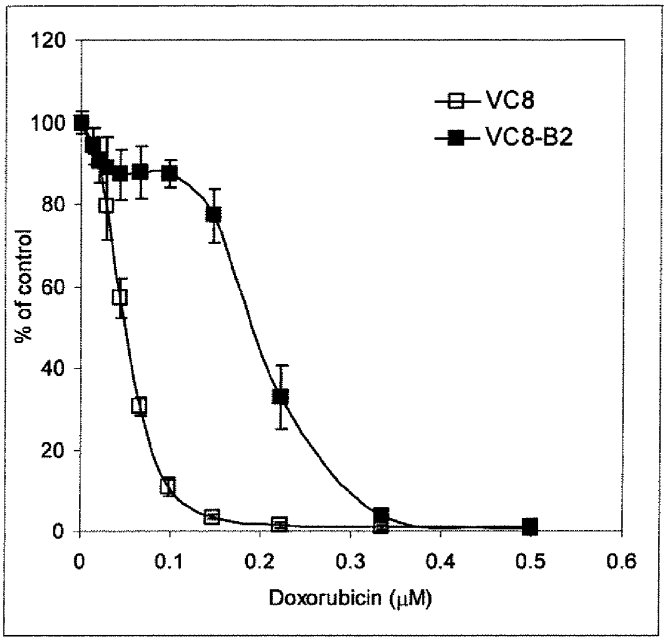




Printed by Jouve, 75001 PARIS (FR)

## Description

### 1. RELATED APPLICATIONS

This application claims priority to U.S. provisional application nos. 61/156,449, filed February 27, 2009 and 61/170,013, filed April 16, 2009. The disclosures of the above referenced applications are incorporated by reference herein in their entireties

### 2. FIELD

Provided herein are methods for treatment of a cancer in a subject having reduced BRCA2 activity. In certain embodiments, the methods comprise administering a therapeutically effective amount of SNS-595 to the subject.

### 3. BACKGROUND

Cancer is one of the leading causes of death in the United States. Each year, more than half a million Americans die from cancer, and more than one million are newly diagnosed with the disease. Cancerous tumors can result when a cell escapes from its normal growth regulatory mechanisms and proliferates in an uncontrolled fashion. Tumor cells can metastasize to secondary sites if treatment of the primary tumor is either not complete or not initiated before substantial progression of the disease. Early diagnosis and effective treatment of tumors can be essential for survival.

Proteins encoded by the breast cancer susceptibility genes (BRCA1 and BRCA2 proteins) have been implicated in predispositions to breast, ovarian, and other cancers. These proteins are expressed and are implicated them in many processes fundamental to all cells including DNA repair and recombination, checkpoint control of cell cycle, and transcription.

BRCA1 and BRCA2 are important for DNA double-strand break (DSB) repair by homologous recombination (HR). Mutations in BRCA1 and BRCA2 genes can predispose individuals to various cancers. Germ-line mutations in BRCA1 and BRCA2 are responsible for approximately 5-10% of all epithelial ovarian cancers (*see,* Li and Karlan, Curr Oncol Rep, 2001 3:27-32). Germ-line mutations in either of these genes have been shown to account for 20-60% of breast cancer cases in families where multiple individuals are affected (about 2-6% of all cases). Nathanson et al., Nature Med, 2001 7, 552-556, 2001. Carriers of BRCA1 and BRCA2 mutations are also susceptible to cancers of prostate, pancreas, and male breast. *See,* Venkitaraman, J. Cell Sci, 2001 114, 3591-98.

In view of the importance of mutations of BRCA1 and BRCA2 in breast, ovarian cancers and other cancers, there is a need for methods for treatment of cancer subjects having BRCA2 mutations or in whom BRCA2 activity is otherwise reduced.

### 4. SUMMARY

It has been observed that presence of a BRCA2 mutation increases responsiveness of cancer cells in a subject to treatment by SNS-595, which is (+)-1,4-dihydro-7-[(3S,4S)-3-methoxy-4-(methylamino)-1-pyrrolidinyl]-4-oxo-1-(2-thiazolyl)-1 ,8-naphthyridine-3-carboxylic acid. Accordingly, provided in one embodiment is a method for treating a cancer subject, such as a cancer patient having a BRCA2 mutation that impairs activity of BRCA2 (or in whose cells BRCA2 activity is down-regulated or reduced relative to normal, e.g., reduced expression), comprising administering to the subject a therapeutically effective amount of SNS-595.

In certain embodiments, the methods provided herein comprise diagnosing a BRCA2 mutation in a cancer subject and treating the subject with SNS-595. In certain embodiments, the methods provided herein comprise diagnosing a BRCA2 activity down-regulation or reduction in a cancer subject and treating the subject with SNS-595.

In certain embodiments, the methods provided herein comprise administering a dose of about 10-100 $mg/m^2$ of SNS-595 to the cancer subject having a BRCA2 mutation or in whom BRCA2 activity (or expression) is reduced.

In certain embodiments, methods provided herein comprise contacting a cancer cell having a BRCA2 mutation with an amount of SNS-595 effective to induce double-strand DNA breaks.

The methods provided herein encompass treatment of breast, ovarian, prostate, pancreas and other cancers wherein a cancer cell exhibits a BRCA2 mutation.

In another aspect, provided herein is a method of identifying a subject for treatment with SNS-595, comprising diagnosing a BRCA2 mutation in the subject.

In certain embodiments, SNS-595 is used alone, i.e., without other chemotherapeutic agents.

In other embodiments, SNS-595 is administered in combination with one or more therapeutic agents, i.e., pharmaceutical agents with activity against cancer or its symptoms. Examples of therapies within the scope of the methods include, but are not limited to, surgery, chemotherapy, radiation therapy, hormonal therapy, biological therapy,

immunotherapy, and combinations thereof. The combinations encompass simultaneous as well as sequential administration.

In some embodiments, the additional therapeutic agent is selected from alkylating agents, antimetabolites, aurora kinase inhibitors, purine antagonists, pyrimidine antagonists, spindle poisons, mitotic inhibitors, topoisomerase II inhibitors and poisons, topoisomerase I inhibitors, anti-neoplastic antibiotics, nitrosoureas, inorganic ion complexes, enzymes, hormones and hormone analogs, EGFR inhibitors, antibodies and antibody derivatives, IMIDs, HDAC inhibitors, Bcl-2 inhibitors, VEGF-stimulated tyrosine kinase inhibitors, VEGFR inhibitors, proteasome inhibitors, cyclin-dependent kinase inhibitors, PARP inhibitors, aromatase inhibitors, and dexamethasone.

In a particular embodiment, the combination therapy comprises administering SNS-595 and at least one therapeutic agent selected from docetaxel, taxotere, vinorelbine, capecitabine, doxorubicin, goserelin, zoledronic acid, paclitaxel, pamidronate, anastrozole, exemestane, cyclophosphamide, epirubicin, fulvestrant, letrozole, gemcitabine, leuprolide, filgrastim, G-CSF or granulocyte colony stimulating factor, pegfilgrastim, toremifene, tamoxifen, bevacizumab, trastuzumab, 5-fluorouracil, methotrexate, trabectidin epoetin alfa, and darbepoetin alfa. In another embodiment, the combination therapy comprises administering SNS-595 and a support care agent.

Also provided are dosing regimens, dosing schedules and methods of using SNS-595 in cancer subjects having impaired BRCA2 activity.

In certain embodiments, provided herein are pharmaceutical compositions comprising SNS-595 and a pharmaceutically acceptable carrier, adjuvant or diluent for treatment of the cancer subjects having reduced BRCA2 activity.

## 5. BRIEF DESCRIPTION OF DRAWINGS

FIG. 1 illustrates inhibition of growth in Chinese hamster cells that are mutant for BRCA2 (VC8) and complemented for functional BRCA2 (VC8-B2) in the presence of doxorubicin.

FIG. 2 illustrates inhibition of growth in VC8 and VC8-B2 cells in the presence of SNS-595.

FIG. 3 illustrates colony outgrowth in human sarcoma U-20S cells (wild-type cells and cells depleted for BRCA2 using siRNA) upon treatment with doxorubicin.

FIG. 4 illustrates colony outgrowth in human sarcoma U-20S cells (wild-type cells and cells depleted for BRCA2 using siRNA) upon treatment with SNS-595.

FIG. 5 provides Pulsed field gel electrophoresis (PFGE) for an 18-hour run for treatment with SNS-595, and doxorubicin, each alone and in co-treatment with aphidicolin, for 240 seconds switch time.

FIG. 6 provides PFGE for a 24-hour run for treatment with SNS-595 and doxorubicin, each alone and in co-treatment with aphidicolin, for 60 to 240 seconds switch time.

FIG. 7 illustrates differences in the DNA-damaging activity of SNS-595 and doxorubicin as demonstrated by the production of more small DNA fragments following treatment with doxorubicin than with SNS-595 for an 18-hour PFGE run.

FIG. 8 illustrates differences in the DNA-damaging activity of SNS-595 and doxorubicin as demonstrated by the production of more small DNA fragments following treatment with doxorubicin than with SNS-595 for a 24-hour PFGE run.

FIG. 9 illustrates cloning efficiency of SPD8 cells upon treatment with each of aphidicolin, SNS-595, and doxorubicin, and with each of SNS-595 and doxorubicin in co-treatment with aphidicolin.

FIG. 10 illustrates HPRT mutation reversion frequency of SPD8 cells upon treatment with aphidicolin, SNS-595, and doxorubicin, and with each of SNS-595 and doxorubicin in co-treatment with aphidicolin.

## 6. DETAILED DESCRIPTION

### 6.1 DEFINITIONS

Unless defined otherwise, all technical and scientific terms used herein have the same meaning as is commonly understood by one of ordinary skill in the art. All patents, applications, published applications and other publications are incorporated by reference in their entirety. In the event that there is a plurality of definitions for a term herein, those in this section prevail unless stated otherwise.

As used herein, "SNS-595" means (+)-1,4-dihydro-7-[(3S,4S)-3-methoxy-4-methylamino-1-pyrrolidinyl]-4-oxo-1-(2-thiazolyl)-1,8-naphthyridine-3-carboxylic acid, as well as any ionic form, salts, solvates, e.g., hydrates, or other forms of that compound, including mixtures thereof. Thus, compositions comprising SNS-595 may include (+)-1,4-dihydro-7-[(3S,4S)-3-methoxy-4-methylamino-1-pyrrolidinyl]-4-oxo-1-(2-thiazolyl)-1,8-naphthyridine-3-carboxylic acid or an ionic form thereof, salt, solvate, e.g., hydrate, or other form of the compound. In some embodiments, SNS-595 is

provided as a pharmaceutically acceptable salt. In certain embodiments, SNS-595 encompasses a composition consisting essentially of (+)-1,4-dihydro-7-[(3S,4S)-3-methoxy-4-methylamino-1-pyrrolidinyl]-4-oxo-1-(2-thiazolyl)-1,8-naphthyridine-3-carboxylic acid, including less than 0.5% (by mass) of other compounds or impurities based on total weight of the composition. Such impurities include compounds having a thiazolyl-oxo-naphthyridine-3-carboxylic acid scaffold, such as (+)-1 ,4-dihydro-7-[(3*S*,4*S*)-hydroxy-4-methylamino-1-pyrrolidinyl]-4-oxo-1-(2-thiazolyl)-1,8-naphthyridine-3-carboxylic acid, (+)-1,4-dihydro-7-[(3*S*,4*S*)-3-methoxy-4-amino-1-pyrrolidinyl]-4-oxo-1-(2-thiazolyl)-1,8-naphthyridine-3-carboxylic acid and/or (+)-1,4-dihydro-7-[(3*S*,4*S*)-3-hydroxy-4-amino-1-pyrrolidinyl]-4-oxo-1-(2-thiazolyl)-1,8-naphthyridine-3-carboxylic acid. Exemplary SNS-595 compositions are described in US Provisional Application No. 61/141,856, the entirety of which is incorporated herein by reference.

As used herein, "enantiomerically pure SNS-595" refers to SNS-595 that is substantially free from (-)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid. In other words, enantiomerically pure SNS-595 is enantiomeric excess of the "(-)" form. The term "enantiomerically pure" or "pure enantiomer" denotes that the compound comprises more than about 95%, 96%, 97%, 98%, 99%, 99.5, 99.6%, 99.7%, 99.8%, or 99.9% by weight of SNS-595.

As used herein, and unless otherwise indicated, the terms "treat," "treating" and "treatment" refer to alleviating or reducing the severity of a disease or a symptom associated with the disease or condition being treated.

As used herein, "prevent", "prevention" and other forms of the word include the inhibition of onset or progression of a disease or disorder or a symptom of the particular disease or disorder. In some embodiments, subjects with familial history of cancer are candidates for preventive regimens. Generally, in the context of cancer, the term "preventing" refers to administration of the drug prior to the onset of signs or symptoms of a cancer, particularly in subjects at risk of cancer.

As used herein, and unless otherwise indicated, the term "managing" encompasses preventing the recurrence of the particular disease or disorder in a subject who had suffered from it, lengthening the time a subject who had suffered from the disease or disorder remains in remission, reducing mortality rates of the subjects, and/or maintaining a reduction in severity or avoidance of a symptom associated with the disease or condition being managed.

As used herein, "subject" means an animal, typically a mammal, including a human being. As used herein, "patient" means a human subject.

As used herein, "sample" or "biological sample" refers to a tissue or an extract, including a cell and physiological fluid from which information can be obtained regarding the BRCA2 status of a subject through methods of analysis known in the art. Typically, the biological sample will be a blood sample. However, a sample may be collected from one or more of a variety of sources from a subject, including body fluid samples, or tissue samples. Suitable tissue samples include various types of tumor or cancer tissue, organ tissue, such as those taken at biopsy. The sample can be treated prior to use, such as preparing plasma from blood, diluting viscous fluids, and the like. Methods of treating a sample can involve filtration, distillation, extraction, concentration, inactivation of interfering components, the addition of reagents, and the like.

As used herein, and unless otherwise specified, the terms "therapeutically effective amount" and "effective amount" of a compound refer to an amount sufficient to provide a therapeutic benefit in the treatment, prevention and/or management of a disease, to delay or minimize one or more symptoms associated with the disease or disorder to be treated. The terms "therapeutically effective amount" and "effective amount" can encompass an amount that improves overall therapy, reduces or avoids symptoms or causes of disease or disorder or enhances the therapeutic efficacy of another therapeutic agent.

As used herein and unless otherwise indicated, the term "pharmaceutically acceptable salt" includes, but is not limited to, a salt of an acidic or basic group that can be present in the compounds provided herein. Under certain acidic conditions, the compound can form a wide variety of salts with various inorganic and organic acids. The acids that can be used to prepare pharmaceutically acceptable salts of such basic compounds are those that form salts comprising pharmacologically acceptable anions including, but not limited to, acetate, benzenesulfonate, benzoate, bicarbonate, bitartrate, bromide, calcium edetate, camsylate, carbonate, chloride, bromide, iodide, citrate, dihydrochloride, edetate, edisylate, estolate, esylate, fumarate, gluceptate, gluconate, glutamate, glycollylarsanilate, hexylresorcinate, hydrabamine, hydroxynaphthoate, isethionate, lactate, lactobionate, malate, maleate, mandelate, methanesulfonate (mesylate), methylsulfate, muscate, napsylate, nitrate, pantothenate, phosphate/diphosphate, polygalacturonate, salicylate, stearate, succinate, sulfate, tannate, tartrate, teoclate, triethiodide, and pamoate. Under certain basic conditions, the compound can form base salts with various pharmacologically acceptable cations. Non-limiting examples of such salts include alkali metal or alkaline earth metal salts and, particularly, calcium, magnesium, sodium, lithium, zinc, potassium and iron salts.

As used herein and unless otherwise indicated, the term "hydrate" means SNS-595 or a salt thereof, further including a stoichiometric or non-stoichiometric amount of water bound by non-covalent intermolecular forces. The hydrates of SNS-595 can be crystalline or non-crystalline.

As used herein and unless otherwise indicated, the term "solvate" means a solvate formed from the association of one or more solvent molecules to a compound provided herein. The term "solvate" includes hydrates (*e.g.*, monohydrate, dihydrate, trihydrate, tetrahydrate, and the like). The solvates of SNS-595 can be crystalline or non-crystalline.

As used herein, the transitional phrase "consisting essentially of' limits the scope of a claim to the specified materials and additional materials do not materially affect the basic and novel characteristic(s) of the claimed subject matter.

The terms "co-administration" and "in combination with" include the administration of two or more therapeutic agents (for example, SNS-595 or a composition provided herein and another anti-cancer agent or other active agent) either simultaneously, concurrently or sequentially with no specific time limits. In one embodiment, SNS-595 and at least one other agent are present in the cell or in the subject's body at the same time or exert their biological or therapeutic effect at the same time. In one embodiment, the therapeutic agent(s) are in the same composition or unit dosage form. In another embodiment, the therapeutic agent(s) are in separate compositions or unit dosage forms.

The term "supportive care agent" refers to any active agent that treats, prevents, manages, reduces, or avoids an adverse or unwanted effect of treatment with SNS-595 alone or in combination with other therapeutic agents. Examples are described herein.

## 6.2 SNS-595

The compound for use in the methods provided herein, including the combination therapy, and in compositions provided herein is enantiomerically pure (+)-1,4-dihydro-7-[(3S,4S)-3-methoxy-4-(methylamino)-1-pyrrolidinyl]-4-oxo-1-(2-thiazolyl)-1 ,8-naphthyridine-3-carboxylic acid, which is also known as SNS-595 or AG-7352. The name assigned by the United States Adopted Names Council (USANC) to the compound is "voreloxin" .

SNS-595 has the following chemical structure:

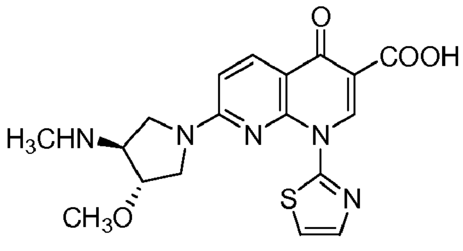

.

In certain embodiments, pharmaceutically acceptable salts, solvates, hydrates or prodrugs of SNS-595 are used in the methods and compositions provided herein.

In certain embodiments, SNS-595 is administered as a composition consisting essentially of enantiomerically pure (+)-1,4-dihydro-7-[(3*S*,4*S*)-3-methoxy-4-methylamino-1 -pyrrolidinyl] -4-oxo-1 -(2-thiazolyl)-1,8-naphthyridine-3 -carboxylic acid, including less than 0.5% (by mass) of other compounds or impurities based on total weight of the composition. Such impurities include compounds having a thiazolyl-oxo-naphthyridine-3-carboxylic acid scaffold, such as (+)-1,4-dihydro-7-[(3*S*,4*S*)-hydroxy-4-methylamino-1 -pyrrolidinyl] -4-oxo-1 -(2-thiazolyl)-1,8-naphthyridine-3 -carboxylic acid, (+)-1,4-dihydro-7-[(3*S*,4*S*)-3-methoxy-4-amino-1-pyrrolidinyl]-4-oxo-1-(2-thiazolyl)-1,8-naphthyridine-3-carboxylic acid and/or (+)-1,4-dihydro-7-[(3*S*,4*S*)-3-hydroxy-4-amino-1-pyrrolidinyl]-4-oxo-1-(2-thiazolyl)-1,8-naphthyridine-3-carboxylic acid.

SNS-595 can be prepared by methods known to one of skill in the art, for example, according to the procedure described in US Application No. 12/650,390, filed December 30, 2009; International Publication No. WO 2007/146335; U.S. Patent No. 5,817,669; and Japanese Patent Application No. Hei 10-173986, the entireties of which are incorporated herein by reference. Certain exemplary pharmaceutical compositions comprising SNS-595 and methods of using the same are described in U.S. Patent Application Pub. Nos. 2005/0203120; 2005/0215583; 2006/0025437; 2006/0063795, 2006/0247267, and US Provisional Application Nos. 61/240,161, filed September 4, 2009, 61/240,113, filed September 4, 2009 and 61/288,213, filed December 18, 2009 which are incorporated herein by reference in their entireties.

## 6.3 METHODS OF TREATMENT

The process of homologous recombinational repair (HRR) is a major DNA repair pathway that acts on double-strand breaks (DSB) and interstrand crosslinks. HRR provides a mechanism for the error-free removal of damage present in DNA that has replicated. Thus, HRR acts in a critical way, in coordination with the S and G2 checkpoint machinery, to eliminate chromosomal breaks before the cell division occurs. *See,* Thompson et al., Mutation Research/Fundamental and Molecular Mechanisms of Mutagenesis, 2001, 477, 131-153.

Cancer cells with BRCA2 mutations that impair BRCA2 activity can have compromised homologous recombination repair. While not intending to be bound by any particular theory of operation, such cells can have greater sensitivity

to treatment with SNS-595 because SNS-595 treatment can result in an increase in the number of double-strand DNA breaks that cannot be repaired because of the deficient HRR in these cells. Likewise, if BRCA2 activity in the cells is reduced compared to normal levels, such as by having down-regulated BRCA2 expression, such cells may have higher sensitivity to SNS-595. Thus, the methods provided herein encompass treating, preventing, or managing cancer in a subject, such as a patient having a BRCA2 mutation by administering a therapeutically effective amount of SNS-595. In certain embodiments, the methods encompass treatment of breast, ovarian, prostate, pancreas and other cancers in a subject, such as a patient that exhibits a BRCA2 mutation. In one embodiment, the cancer is breast cancer in a subject, such as a patient that exhibits a BRCA2 mutation.

In certain embodiments, the methods encompass treatment of breast, ovarian, prostate, pancreas and other cancers where a cancer cell exhibits a BRCA2 mutation. In one embodiment, the cancer is breast cancer where a cancer cell exhibits a BRCA2 mutation.

In another aspect, provided herein is a method of identifying a subject for treatment with SNS-595, comprising diagnosing a BRCA2 mutation in the subject.

In certain embodiments, methods provided herein comprise contacting a cancer cell in or from a subject, such as a patient, having a BRCA2 mutation with a therapeutically effective amount of SNS-595. In general, the amount of SNS-595 employed is effective to induce double-strand DNA breaks. The contacting can be *in vitro, in vivo,* or *ex vivo.* In one embodiment, the method comprises contacting a cancer cell *in vivo.*

In one embodiment, provided herein is a method of identifying a cancer subject suitable for treatment with SNS-595, comprising: (a) obtaining a biological sample from a candidate having cancer; (b) screening the biological sample for a BRCA2 mutation; and (c) if the candidate has a BRCA2 mutation, identifying the candidate as a cancer subject suitable for treatment with SNS-595. In another embodiment, the candidates identified as cancer subjects suitable for treatment with SNS-595 are treated with SNS-595.

In another embodiment, provided herein is a method for identifying one or more cancer subjects suitable for treatment with SNS-595 from a plurality of candidate cancer subjects. The method comprises identifying one or more cancer subjects having a BRCA2 mutation from the plurality as cancer subjects suitable for treatment with SNS-595. In one embodiment, one or more suitable subjects are treated with SNS-595.

In one embodiment, one or more subjects whose BRCA2 activity levels are in the normal range (or not significantly reduced) are not treated with SNS-595. In certain embodiments, all subjects whose BRCA2 activity levels are in the normal range (or not significantly reduced) are not treated with SNS-595. For example, a subject whose BRCA2 activity level is normal (or not significantly reduced) may be treated using an alternative therapeutic modality, such as a chemotherapeutic whose anticancer activity is not affected by BRCA2 status. Alternatively, a subject having normal or near-normal BRCA2 activity may be treated with SNS-595 using a different treatment protocol, such as a treatment schedule in which doses of SNS-595 are administered more frequently or higher doses of SNS-595 are used than would be considered for the patient in which BRCA2 activity is reduced.

In one embodiment, one or more subjects who do not show a BRCA2 mutation are not treated with SNS-595. In certain embodiments, all subjects who do not show a BRCA2 mutation are not treated with SNS-595. For example, a subject not having a BRCA2 mutation may be treated using an alternative therapeutic modality, such as a chemotherapeutic whose anticancer activity is not affected by a BRCA2 mutation. Alternatively, a subject not having a BRCA2 mutation may be treated with SNS-595 using a different treatment protocol, such as a treatment schedule in which doses of SNS-595 are administered more frequently or higher doses of SNS-595 are used than would be considered for the patient having a BRCA2 mutation.

The BRCA2 mutation in a cancer cell can be diagnosed by any suitable technique known in the art, such as gene sequencing, including BRACAnalysis® test from Myriad Genetic Laboratories, Inc., multiplex ligation-dependent probe amplification (MLPA), high-resolution melt curve analysis (*see,* Dufresne et al., Arch Pathol Lab Med, 2006,130:185-187 and Takano et al., BMC Cancer, 2008, 8:59), protein truncation test (PTT), denaturing gradient gel electrophoresis (DGGE), and/or denaturing high pressure liquid chromatography (DHPLC).

In certain embodiments, the mutations in BRCA2 are small deletions or insertions, which can be found along the whole protein. The methods provided herein encompass treatment of cancer subjects with any BRCA2 mutation known to one of skill in the art. Examples of such mutations include, for example, 999del5, 6174delT, 8803delC, 4486de1G, 5445del5 and 2024del5, 763insAT, 763insAT, 983delACAG, A3058T, 3758delACAG, 3908delTG, 4706delAAAG, 5804delTTAA, C6137A, 6174delT, 6305insA, 9132de1C, de12352ins12, dup9700, de11518, and others as described by, for example, Loman et al., J Natl Cancer Inst, 2001, 93(16):1215-1223; Peto et al., J Natl Cancer Inst, 1999, 91(11):943-949; and Walsh et al., JAMA, 2006, 295(12):1379-88.

Without being bound to any particular theory, it is believed that SNS-595 induces site-selective DNA damage by selectively intercalating DNA and poisoning topoisomerase II, resulting in replication-dependent DNA damage, irreversible G2 arrest and rapid apoptosis. In contrast, anthracycline-based therapies induce topoisomerase II-mediated DNA DSBs as well as DNA-damaging activity through non-topoisomerase II associated mechanisms, including the generation of DNA adducts, formation of DNA crosslinks and the production of reactive oxygen species (ROS). *See,*

Gewirtz D., Biochem Pharmacol 1999, 57:727-41.

The targeted DNA-enzyme interactions in SNS-595 therapy stand in contrast with the mechanistically more promiscuous and highly intercalative anthracyclines that are in clinical use (O'Reilly et al., Biochemistry 2002, 41:7989-97; Wang J., Nat Rev Mol Cell Biol, 2002, 3:430-40). These differentiating features indicate that the role of BRCA2 in the repair of SNS-595-induced DNA damage is not directly transportable from data pertaining to anthracyclines, such as doxorubicin, or topoisomerase II inhibitors such as, etoposide. The data provided in the examples section demonstrating sensitization of BRCA2 mutant cells to SNS-595 confirms the role of BRCA2 in the repair of SNS-595-induced DNA damage.

In certain embodiments, the methods of treatment provided herein comprise administering a dose of about 10-100 $mg/m^2$ of SNS-595 to the subject. In certain embodiments, the methods of treatment comprise administering a dose of about 10-100 $mg/m^2$, about 20-90 $mg/m^2$, about 30-90 $mg/m^2$, about 40-90 $mg/m^2$, about 30-80 $mg/m^2$, about 40-80 $mg/m^2$, or about 30-50 $mg/m^2$ to the subject.

In some embodiments, the methods comprise administering to the subject a therapeutically effective amount of SNS-595 in combination with a therapeutically effective amount of a second active agent. In some embodiments, the second active agent is a therapeutic antibody to a cancer antigen, a hematopoietic growth factor, a cytokine, an anti-cancer agent, an antibiotic, a cox-2 inhibitor, an immunomodulatory agent, an immunosuppressive agent, a corticosteroid, or a pharmacologically active mutant or derivative thereof. In other embodiments, the second active agent is an alkylating agent, an anti-neoplastic antibiotic, an anti-metabolite, a platinum coordination complex, a topoisomerase II inhibitor or poison, a CDK inhibitor, an aurora kinase inhibitor, a purine antagonist, a pyrimidine antagonist, a spindle poison, a mitotic inhibitor, a topoisomerase I inhibitor, a nitrosourea, an inorganic ion complex, an enzyme, a hormone or hormone analog, an EGFR inhibitor, an antibody or antibody derivative, an IMID, an HDAC inhibitor, a Bcl-2 inhibitor, a VEGF-stimulated tyrosine kinase inhibitor, a VEGFR inhibitor, a proteasome inhibitor, an aromatase inhibitor, a PARP inhibitor, dexamethasone, or radiation.

### 6.3.1 SUBJECTS

In certain embodiments of the methods provided herein, the subject to be treated is an animal, for example a mammal or a non-human primate. In particular embodiments, the subject is a human patient. The subject can be male or female.

Particularly, subjects amenable to treatment according to the methods provided herein include subjects with cancer of the breast, ovary, pancreas, or prostate, and have reduced BRCA2 activity. In a particular embodiment, the subject suffers from breast cancer. In certain embodiments, breast cancer is refractory to and/or relapsed from prior therapy. The reduction in BRCA2 activity may be associated with any BRCA2 mutation known to those of skill in the art. In many embodiments, the BRCA2 mutation is a mutation that impairs the activity of BRCA2, which can manifest in various mechanistic forms. For example, the mutation may impair the expression of BRCA2, i.e., the amount of BRCA2 protein produced in cells, or the mutation may impair a biological activity of the protein, such as an interaction of BRCA2 with another protein or a nucleic acid or an enzymatic activity of the BRCA2 protein. The BRCA2 mutation, or deficiency of activity, can be diagnosed in the subject by any technique deemed suitable by one of skill in the art. Exemplary techniques are described in Oncogene, 1998, 16(23): 3069-82, Kuznetsov et al., Nature Medicine, 2008, 14, 875-881, Dufresne et al., Arch Pathol Lab Med, 2006,130:185-187 and Takano et al., BMC Cancer, 2008, 8:59.

Also encompassed are methods of treating a subject regardless of the subject's age, although some diseases or disorders are more common in certain age groups. In some embodiments, the subject is a human patient at least 18 years old. In some embodiments, the patient is 10, 15, 18, 21, 24, 35, 40, 45, 50, 55, 65, 70, 75, 80, or 85 years old or older.

In some embodiments, the methods find use in patients at least 50 years of age, although younger patients could benefit from the method as well. In other embodiments, the patients are at least 55, at least 60, at least 65, and at least 70 years of age. In certain embodiments, the methods provided herein are useful in a female patient who has a personal history of early-onset (before age 50 years) breast cancer or early-onset breast and ovarian cancer at any age. In another embodiment, the patient is a female and has a family history of breast cancer or breast and ovarian cancer. In another embodiment, the patient is a male with a personal or family history of male breast cancer.

In certain embodiments, the methods provided herein encompass the treatment of subjects who have not been previously treated for cancer. In other embodiments, the methods encompass treating subjects who have been previously treated but are non-responsive to standard therapies as well as those who are currently being treated for cancer. For example, the subjects may have been previously treated or are currently being treated with a standard treatment regimen for cancer known to the practitioner of skill in the art.

In some embodiments, the subject has not previously undergone treatment with SNS-595. In some embodiments, the subject has previously undergone treatment with SNS-595.

### 6.3.2 DOSAGES

In certain representative embodiments, the method of treating, preventing or managing cancers provided herein comprises administering to a subject an effective amount of SNS-595 via any acceptable route of administration. In general, the method would comprise administering to the subject, on the basis of body surface area, a dose of about 10-100 $mg/m^2$ of SNS-595. In another embodiment, the method of comprises administering a dose of about 20-90 $mg/m^2$ of SNS-595. In another embodiment, the method comprises administering a dose of about 40-90 $mg/m^2$ of SNS-595. In another embodiment, the method comprises administering a dose of about 30-50 $mg/m^2$ of SNS-595. In another embodiment, the method comprises administering a dose of about 30-90 $mg/m^2$ of SNS-595. In another embodiment, the method comprises administering a dose of about 40-90 $mg/m^2$ of SNS-595. In another embodiment, the method comprises administering a dose of about 30-80 $mg/m^2$ of SNS-595. In another embodiment, the method comprises administering a dose of about 40-80 $mg/m^2$ of SNS-595.

In one embodiment, SNS-595 is administered intravenously and in an amount of about 10, 15, 18, 21, 24, 25, 27, 30, 35, 40, 45, 48, 50, 55, 60, 63, 70, 72, 75, 80, 85, 90, 95, or 100 $mg/m^2$ (which amount may be provided in single or divided doses) in one day.

The skilled practitioner in treating cancer typically employs a dosage unit that enables approximation of the subject's exposure to the active ingredient being administered. Any suitable dosage unit may be employed.

For example, the dosage unit used may approximate exposure based on a calculation of body surface area. Body surface area (BSA) calculations for a human subject can be calculated, for example, using the Mosteller formula:

$$\text{BSA } (m^2) = [(\text{height (cm)} \times \text{body mass (kg)} / 3600]^{1/2}.$$

The most common such dosage unit is milligrams of active compound per square meter of body surface area ($mg/m^2$).

As another example, the administered dose of the SNS-595 can be expressed in units other than $mg/m^2$. For example, doses can be expressed as milligrams of active compound per kilogram of body mass (mg/kg). One of ordinary skill in the art would readily know how to convert a patient dose from $mg/m^2$ to mg/kg, given the height and/or body mass of the patient (*see,* http://www.fda.gov/cder/cancer/animalframe.htm). For example, a dose of 1-30 $mg/m^2$ for a 65 kg human is approximately equal to 0.026-0.79 mg/kg. Other dosage units may also be employed.

In certain embodiments, the administered dose of SNS-595 can be delivered as a single bolus *(e.g.,* intravenous injection) or over a longer period *(e.g.,* continuous infusion or periodic bolus doses). Administration of SNS-595 may be repeated until the subject experiences stable disease or regression or until the subject experiences disease progression or unacceptable toxicity. Stable disease or lack thereof is determined by methods known in the art, such as evaluation of symptoms, physical examination, and other commonly accepted parameters.

The amount of SNS-595 administered according to the methods provided herein will depend on various factors, such as the overall health of the subject being treated, the severity of the disorder or symptom of the disorder, the active ingredient being administered, the manner of administration, the frequency of administration, other medications present, and the judgment of the prescribing physician. The amount to be administered can be empirically determined by the physician.

In some embodiments, the frequency of administration is in the range of about a daily dose to about a monthly dose. In certain embodiments, administration is once per day, once every other day, 3 days in a row, 4 days in a row, on days 1 and 4, on days 1 and 2, on days 1 and 3, once per week, twice per week, three times per week, once every two weeks, once every three weeks, or once every four weeks. In one embodiment, the pharmaceutical composition provided herein is administered once per week for three weeks. In another embodiment, the pharmaceutical composition provided herein is administered once every three weeks. In one embodiment, the pharmaceutical composition provided herein is administered once every three weeks. In another embodiment, the pharmaceutical composition provided herein is administered once every four weeks.

In certain embodiments, SNS-595 is administered to a subject in one or more cycles of administration. Cycling therapy involves the administration of one or more doses of SNS-595, followed by a period of rest, and repeating this administration/rest cycle. Cycling therapy can reduce the development of resistance to one or more of the therapies, avoid or reduce the side effects of one or more of the therapies, and/or improve the efficacy or duration of the treatment.

Consequently, in one embodiment, a dose of SNS-595 is administered once per week, , in a three- to six-week cycle with a rest period of about 1 to about 30 days between doses. In some embodiments, the waiting period is 14 days, with the first dose given on day 1 and the next dose given on day 15. Treatment in such cases may thus be said to be using a "14-day cycle." In some embodiments, the doses may be given 28 days apart, i.e., a 28-day cycle.

In another embodiment, the dosing method comprises a cycle wherein the cycle comprises administering a dose of SNS-595 to a subject once per week for three weeks followed by a period of at least 14 days in which no compound or composition is administered to the subject and wherein the cycle is repeated a plurality of times. In another

embodiment, the period in which no compound or composition is administered is 18 days. In another embodiment, the period in which no compound or composition is administered is 21 days. In another embodiment, the period in which no compound or composition is administered is 28 days. The frequency, number and length of dosing cycles can be increased or decreased.

In one embodiment, the method provided herein comprises: i) administering a dose of SNS-595, *e.g.*, about 40-90 mg/$m^2$, to a subject; ii) waiting a period of at least six days where the subject is not administered any SNS-595; and iii) administering another dose of SNS-595, *e.g.*, about 40-90 mg/$m^2$, of SNS-595 to the subject. In one embodiment, steps ii)-iii) are repeated a plurality of times.

In one embodiment, the method provided herein comprises: i) administering a dose of SNS-595, e.g., about 30-50 mg/$m^2$, to a subject; ii) waiting a period of at least six days in which the subject is not administered any SNS-595; and iii) administering another dose of SNS-595, *e.g.*, about 30-50 mg/$m^2$, of SNS-595 to the subject. In one embodiment, steps ii)-iii) are repeated a plurality of times.

In another embodiment, the method comprises administering a dose of about 40 mg/$m^2$, about 45 mg/$m^2$, about 48 mg/$m^2$, about 50 mg/$m^2$, about 60 mg/$m^2$, about 72 mg/$m^2$, about 75 mg/$m^2$, about 80 mg/$m^2$, or about 90 mg/$m^2$ of SNS-595, in each of the foregoing steps i) and iii).

In another embodiment, provided herein is a method for treatment of a breast cancer subject having reduced BRCA2 activity, comprising administering a dose of about 48 mg/$m^2$ of SNS-595 to the subject once every three weeks. In another embodiment, provided herein is a method for treatment of a breast cancer subject having a BRCA2 mutation comprising administering a dose of about 60 mg/$m^2$ of SNS-595 to the subject once every three weeks. In another embodiment, provided herein is a method for treatment of a breast cancer subject having BRCA2 mutation comprising administering a dose of about 75 mg/$m^2$ of SNS-595 to the subject once every three weeks.

In certain embodiments, the dosing method comprises administering to a subject a dose of SNS-595 twice per week for two weeks (dosing on days 1, 4, 8 and 11). In another embodiment, the dosing method comprises administering a once-per-week dose of SNS-595 to a subject. In another embodiment, the dosing method comprises administering a dose of SNS-595 to a subject once every two weeks. In another embodiment, the dosing method comprises administering a dose of SNS-595 to a subject once every three weeks. In another embodiment, the dosing method comprises administering a dose of SNS-595 to a subject once every four weeks.

In one embodiment, a dose of about 40-80 mg/$m^2$ of SNS-595 is administered to a subject once every three weeks wherein the three-week period comprises a treatment cycle and the treatment cycle is repeated at least one time. In another embodiment, the method comprises administering a dose of about 40-80 mg/$m^2$ of SNS-595 to a subject once every four weeks wherein the four-week period comprises a treatment cycle and the treatment cycle is repeated at least one time. In another embodiment, the method comprises administering a dose of about 48 mg/$m^2$ of SNS-595 to a subject once every three weeks wherein the three-week period comprises a treatment cycle and the treatment cycle is repeated at least one time. In another embodiment, the method comprises administering a dose of about 60 mg/$m^2$ of SNS-595 to a subject once every four weeks wherein the four-week period comprises a treatment cycle and the treatment cycle is repeated at least one time. In another embodiment, the method comprises administering a dose of about 75 mg/$m^2$ of SNS-595 to a subject once every four weeks wherein the four-week period comprises a treatment cycle and the treatment cycle is repeated at least one time.

In one embodiment, the method comprises administering a dose of about 40-80 mg/$m^2$ of SNS-595 to a subject once per week wherein the one-week period comprises a treatment cycle, and the treatment cycle is repeated at least twice or at least three times. In another embodiment, the method comprises administering a dose of about 30-50 mg/$m^2$ of SNS-595 to a subject twice per week wherein the one-week period comprises a treatment cycle and the treatment cycle is repeated at least two times. In another embodiment, the dose is about 50 mg/$m^2$ of SNS-595 once per week wherein the one-week period comprises a treatment cycle and the treatment cycle is repeated at least three times. In another embodiment, the dose is about 60 mg/$m^2$ of SNS-595 once per week wherein the one-week period comprises a treatment cycle and the treatment cycle is repeated at least three times. In another embodiment, the dose is about 72 mg/$m^2$ of SNS-595 once per week wherein the one-week period comprises a treatment cycle and the treatment cycle is repeated at least three times. In another embodiment, the method comprises administering a dose of about 40 mg/$m^2$ of SNS-595 to a subject twice per week wherein the one-week period comprises a treatment cycle and the treatment cycle is repeated at least two times.

All methods and dosages described herein apply to the treatment or prevention of cancer or precancerous condition.

### 6.3.3 ADDITIONAL ACTIVE AGENTS

It will also be appreciated that SNS-595 and pharmaceutical compositions comprising SNS-595 can be employed in complementary combination therapies with other active agents or medical procedures.

SNS-595 and pharmaceutical compositions thereof can be administered concurrently with, prior to, or subsequent to, one or more other desired active agents or medical procedures. The particular combination of therapies (agents or procedures) to employ in a combination regimen will take into account compatibility of the desired therapeutics and/or procedures and the desired therapeutic effect to be achieved. It will also be appreciated that the therapies employed may achieve a desired effect for the same disorder (for example, SNS-595 may be administered concurrently with another active agent used to treat the same disorder), or they may achieve different effects (*e.g.*, control of any adverse effects). Non-limiting examples of such agents and procedures include surgery, radiotherapy (*e.g.*, gamma-radiation, neutron beam radiotherapy, electron beam radiotherapy, proton therapy, brachytherapy, and systemic radioisotopes), endocrine therapy, biologic response modifiers (interferons, interleukins, and tumor necrosis factor (TNF) to name a few examples), hyperthermia and cryotherapy, agents to attenuate any adverse effects (*e.g.*, antiemetic agents), and other approved chemotherapeutic anticancer agents.

Examples of chemotherapeutic anticancer agents that may be used as second active agents in combination with SNS-595 include, but are not limited to, alkylating agents (*e.g.*, mechlorethamine, chlorambucil, cyclophosphamide, melphalan, ifosfamide), antimetabolites (*e.g.,* methotrexate), aurora kinase inhibitors (*e.g.,* SNS-314), purine antagonists and pyrimidine antagonists (*e.g.*, 5-fluorouracil (5-FU), gemcitabine), spindle poisons (*e.g.*, vinca alkaloids such as vinblastine, vincristine, vinorelbine), mitotic inhibitors (*e.g.*, taxanes such as paclitaxel, docetaxel, taxotere), topoisomerase II inhibitors or poisons (*e.g.*, epipodophyllotoxins such as etoposide, teniposide; anthracyclines such as doxorubicin, daunorubicin, idarubicin), topoisomerase I inhibitors (*e.g.*, irinotecan, topotecan, camptothecin), anti-neoplastic antibiotics (*e.g.*, bleomycin, mitomycin, aphidicolin; anthracenediones such as mitoxantrone), nitrosoureas (*e.g.*, carmustine, lomustine), inorganic ions (*e.g.*, platinum complexes such as cisplatin, carboplatin, oxaliplatin), enzymes (*e.g.,* asparaginase), hormones and hormone analogs (*e.g.,* tamoxifen, leuprolide, flutamide, megestrol), EGFR (Her1, ErbB-1) inhibitors (*e.g.*, gefitinib), antibodies (*e.g.,* bevacizumab, rituximab), antibody derivatives (*e.g.,* ranibizumab), IMIDs (*e.g.,* thalidomide, lenalidomide), HDAC inhibitors (*e.g.,* vorinostat), Bcl-2 inhibitors (*e.g.,* oblimersen), VEGF-stimulated tyrosine kinase inhibitors (*e.g.*, sorafenib, sunitinib), VEGFR inhibitors (*e.g.,* trastuzumab), proteasome inhibitors (*e.g.,* bortezomib), cyclin-dependent kinase (cdk) inhibitors (*e.g.,* SNS-032, seliciclib), PARP inhibitors (*e.g.,* BSI-201), aromatase inhibitors (*e.g.*, anastrozole, exemestane, letrozole)), trabectidin, and dexamethasone.

In one embodiment, examples of chemotherapeutic anticancer agents that may be used as second active agents in combination with SNS-595 include, docetaxel, vinorelbine, capecitabine, doxorubicin, goserelin, zoledronic acid, paclitaxel, pamidronate, anastrozole, exemestane, cyclophosphamide, epirubicin, fulvestrant, letrozole, gemcitabine, leuprolide, filgrastim (G-CSF or granulocyte colony stimulating factor), toremifene, tamoxifen, anastrozole, dexrazoxane, trastuzumab, pegfilgrastim, epoetin alfa, and darbepoetin alfa. In certain embodiments, SNS-595, in combination with one or more of these therapeutic agents, can be used for the treatment of breast cancer.

In one embodiment, the therapeutic agent is selected from paclitaxel, cisplatin, carboplatin, gemcitabine, topotecan, altretamine, trabectidin, and cyclophosphamide. In certain embodiments, SNS-595, in combination with one or more of these agents, can be used for the treatment of ovarian cancer.

In one embodiment, the second agent is selected from mitoxantrone, prednisone, paclitaxel, docetaxel, estramustine, doxorubicin, goserelin, leuprolide, and degarelix. In certain embodiments, SNS-595, in combination with one or more of these agents, can be used for the treatment of prostate cancer.

Some specific anticancer agents that can be used in combination with SNS-595 include, but are not limited to: carboplatin, cisplatin, gemcitabine, and combinations of any two or more thereof.

In other embodiments, the additional active agent is a supportive care agent, such as an antiemetic agent or a chemoprotectant agent. Specific antiemetic agents include, but are not limited to, phenothiazines, butyrophenones, benzodiazapines, corticosteroids, serotonin antagonists, cannabinoids, and NK1 receptor antagonists. Examples of phenothiazine antiemetic agents include, but are not limited to, prochlorperazine and trimethobenzamide. Examples of butyrophenone antiemetic agents include, but are not limited to, haloperidol. Examples of benzodiazapine antiemetic agents include, but are not limited to, lorazepam. Examples of corticosteroid antiemetic agents include, but are not limited to, dexamethasone. Examples of serotonin receptor (5-HT3 receptor) antagonist antiemetic agents include, but are not limited to, dolasetron mesylate (*e.g.*, Anzemet®), granisetron (*e.g.,* Kytril®), itasetron, ondansetron (*e.g.,* Zofran®), palonosetron (*e.g.,* Aloxi®) ramosetron, tropisetron (*e.g.*, Navoban®), batanopride, dazopride, renzapride. Examples of cannabinoid antiemetic agents include, but are not limited to, dronabinol. Examples of NK1 receptor antagonists include, but are not limited to, aprepitant (*e.g.*, Emend®).

Other supportive care agents include chemoprotectant agents such as amifostine (*e.g.,* Ethyol®), dexrazoxane (*e.g.,* Zinecard®), leucovorin (folinic acid), and mesna (*e.g.*, Mesnex®); thrombopoeitic growth factors such as interleukin-11 (IL-11, oprelvekin, *e.g.,* Neumega®); bisphosphonates such as pamidronate disodium (*e.g.,* Aredia®), etidronate disodium (*e.g.,* Didronel®) and zoledronic acid (*e.g.,* Zometa®); and TNF antagonists, such as infliximab (*e.g.*, Remicade®).

In certain embodiments, administration of SNS-595 is performed in combination with one or more supportive care treatment(s) to mitigate or prevent tumor lysis syndrome or its component symptoms. Treatments suitable for preventing or mitigating TLS (or any of the symptoms thereof, including hyperkalemia, hyperphosphatemia, hyperuricemia, hypocalcemia, and acute renal failure), include, for example, allopurinol (*e.g.,* Zyloprim®), rasburicase (*e.g.,* Elitek®), and sodium polystyrene sulfonate (*e.g.*, Kayexalate®). Leukapheresis may be performed, for example, up to 72 hours after the first treatment with SNS-595.

## 6.4 COMBINATION THERAPY WITH OTHER ACTIVE AGENTS

In certain embodiments, the method provided herein comprises administering SNS-595 or pharmaceutical compositions provided herein in combination with one or more other active agents, and/or in combination with radiation therapy or surgery.

The administration of SNS-595 and the additional active agents to a subject can occur simultaneously or sequentially by the same or different routes of administration. The suitability of a particular route of administration employed for a particular active agent will depend on the active agent itself (*e.g.*, whether it can be administered orally without decomposing prior to entering the blood stream) and the disease being treated. Recommended routes of administration for such other active agents are known to those of ordinary skill in the art. *See, e.g., Physicians' Desk Reference,* (63rd ed., 2009) (hereinafter *"Physicians' Desk Reference*").

In one embodiment, the second active agent is administered intravenously or subcutaneously and once or twice daily in an amount of from about 1 to about 1,000 mg, from about 5 to about 500 mg, from about 10 to about 375 mg or from about 50 to about 200 mg.

In another embodiment, provided herein are methods of treating, preventing and/or managing cancer in a subject having a BRCA2 mutation, which comprise administering SNS-595 in conjunction with (*e.g.*, before, during, or after) conventional therapy including, but not limited to, surgery, immunotherapy, biological therapy, radiation therapy or other non-drug based therapy presently used to treat, prevent or manage cancer.

In one embodiment, SNS-595 can be administered in an amount of about 10-100 $mg/m^2$, about 20-90 $mg/m^2$, about 30-80 $mg/m^2$, about 40-80 $mg/m^2$, about 40-90 $mg/m^2$, about 30-90 $mg/m^2$, or about 30-50 $mg/m^2$, alone or in combination with a second active agent disclosed herein, prior to, during, or after the use of conventional therapy.

In one embodiment, the second agent is selected from the group consisting of carboplatin, cisplatin, gemcitabine, and combinations any two or more thereof.

In one embodiment, the combination therapy comprises administering SNS-595 and carboplatin. In one embodiment, the combination therapy comprises administering SNS-595 and cisplatin. In one embodiment, the combination therapy comprises administering SNS-595 and gemcitabine.

In one embodiment, the methods provided include the administration of SNS-595 in combination with about 5 $mg/m^2$ to about 200 $mg/m^2$ cisplatin. For example, one embodiment includes administration of cisplatin at a dose of about 50 or 70 $mg/m^2$ once every 3 to 4 weeks. One embodiment includes administration of cisplatin at a dose of about 50 or 70 $mg/m^2$ once every 3 weeks. Another embodiment includes administration of cisplatin at a dose of about 75 or 100 $mg/m^2$ once every 3 weeks. In another embodiment, administration of cisplatin is at a dose of about 20 $mg/m^2$ daily for up to 5 days. The administration of cisplatin can be made by intravenous infusion, intravenous push, bolus injection or subcutaneous injection. In one embodiment, the administration of cisplatin is once every 3 to 4 weeks, while the administration of SNS-595 occurs once per week for three weeks or once every three weeks. In one embodiment, the administration of cisplatin is daily for 5 days, while the administration of SNS-595 occurs once per week for three weeks or once every three weeks. In one embodiment, the administration of cisplatin is once a week for 3 weeks, while the administration of SNS-595 occurs once per week for three weeks or once every three weeks.

In one embodiment, the methods provided include the administration of SNS-595 in combination with about 50 $mg/m^2$ to about 400 $mg/m^2$ carboplatin. For example, one embodiment includes administration of carboplatin at a dose of about 300 or about 360 $mg/m^2$ once every 3 weeks. One embodiment includes administration of carboplatin at a dose of about 300 or 360 $mg/m^2$ once every 4 weeks. The administration of carboplatin can be made by intravenous infusion, intravenous push, bolus injection or subcutaneous injection. In one embodiment, the administration of carboplatin is once every 3 weeks, while the administration of SNS-595 occurs once per week for three weeks or once every three weeks. In one embodiment, the administration of carboplatin is once a week for 3 weeks, while the administration of SNS-595 occurs once per week for three weeks or once every three weeks.

In one embodiment, the methods provided include the administration of SNS-595 in combination with about 100 $mg/m^2$ to about 1500 $mg/m^2$ gemcitabine. For example, one embodiment includes administration of gemcitabine at a dose of about 1000 or 1250 $mg/m^2$ once every week for at least 4 weeks. The administration of gemcitabine can be made by intravenous infusion, intravenous push, bolus injection or subcutaneous injection. In one embodiment, the administration of gemcitabine is once a week for up to 4 weeks, while the administration of SNS-595 occurs once per week for three weeks or once every three weeks. In one embodiment, the administration of gemcitabine is twice a week for 2 weeks, while the administration of SNS-595 occurs once per week for three weeks.

In certain embodiments, the second active agent is co-administered with SNS-595 or administered with 1-50 hours delay. In certain embodiments, SNS-595 is administered first followed by administration with the second active

agent with 1-50 hours delay. In other embodiments, the second active agent is administered first followed by administration of SNS-595 with 1-50 hours delay. In some embodiments, the delay is 24 hours.

In another embodiment, the method provided herein comprises: a) administering to a cancer subject having a BRCA2 mutation a dose of about 10-100 mg/$m^2$ of SNS-595 and b) administering to the subject a therapeutically effective amount of a supportive care agent.

The supportive care agent is administered according to the appropriate dosing regimen for that substance. For example, different supportive care agents for treating nausea have different dosing regimen. While some such agents are administered prophylactically, others are co-administered with a compound or composition provided herein while still others are administered after the administration of SNS-595. Illustrative examples of supportive care agents their doses and dosing regimens are found in *Physicians' Desk Reference.* Some exemplary support care agents are disclosed in U.S. Application Publication No. 2006-0025437, the entirety of which incorporated herein by reference.

### 6.5 PHARMACEUTICAL COMPOSITIONS AND DOSAGE FORMS

The methods provided herein use pharmaceutical compositions containing SNS-595 and pharmaceutically acceptable carriers, such as diluents or adjuvants, or in combination with other active ingredient, such as another anti-cancer agent. In clinical practice, SNS-595 may be administered by any conventional route, including but not limited to orally, parenterally, rectally or by inhalation (*e.g.*, in the form of aerosols). Parenteral dosage forms can be administered to subjects by various routes including, but not limited to, subcutaneous, intravenous (including bolus injection), intramuscular, and intraarterial. Because their administration typically bypasses subject's natural defenses against contaminants, parenteral dosage forms are sterile or capable of being sterilized prior to administration to a subject. Examples of parenteral dosage forms include, but are not limited to, solutions ready for injection, dry products ready to be dissolved or suspended in a pharmaceutically acceptable vehicle for injection, suspensions ready for injection, and emulsions. In one embodiment, SNS-595 is administered by an IV injection.

The pharmaceutical compositions for parenteral administration can be emulsions or homogeneous solutions. Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, petroleum oil, oil of animal, vegetable or synthetic origin, such as peanut oil, soybean oil, mineral oil, sesame oil and the like; ethyl oleate, isopropyl myristate, and benzyl benzoate.

These pharmaceutical compositions can also contain adjuvants, in particular wetting, isotonizing, emulsifying, dispersing, and stabilizing agents. Sterilization can be carried out in several ways, for example using a 0.2 micron filter, by radiation or by heating. *See,* Remington's Pharmaceutical Sciences, 21st ed., Mack Publishing, Easton, PA (2005) (hereinafter *"Remington's Pharmaceutical Sciences*"). They can also be prepared in the form of sterile solid pharmaceutical compositions which can be dissolved at the time of use in sterile water or any other injectable sterile medium.

Pharmaceutical compositions can be used in the preparation of individual, single unit dosage forms. Pharmaceutical compositions and dosage forms comprise compound and one or more excipients.

Pharmaceutical compositions and dosage forms can also comprise one or more additional active ingredients. Examples of optional second, or additional, active ingredients are disclosed herein.

In certain embodiments, the pharmaceutical composition provided herein is a single unit dosage form. Pharmaceutical compositions and single unit dosage forms provided herein comprise a prophylactically or therapeutically effective amount of compound or composition, and typically one or more pharmaceutically acceptable carriers or excipients. The term "carrier" refers to a diluent, adjuvant (*e.g.*, Freund's adjuvant (complete and incomplete)), excipient, or vehicle with which the therapeutic is administered. Examples of suitable pharmaceutical carriers are described in *Remington's Pharmaceutical Sciences.*

Typical pharmaceutical compositions and dosage forms comprise one or more excipients. Suitable excipients are well-known to those skilled in the art of pharmacy, and non limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form depends on a variety of factors well known in the art including, but not limited to, the way in which the dosage form will be administered to a subject and the specific active ingredients in the dosage form. The pharmaceutical composition or single unit dosage form, if desired, can also contain minor amounts of wetting or emulsifying agents, or pH buffering agents.

Compounds that increase the solubility of one or more of the active ingredients disclosed herein can also be incorporated into the parenteral dosage forms. For example, cyclodextrin and its derivatives can be used to increase the solubility of active ingredients. *See, e.g.,* U.S. Patent No. 5,134,127, the entirety of which is incorporated herein by reference.

The pH of a pharmaceutical composition or dosage form may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting pharmaceutical composition.

Further provided herein are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical compositions and single unit dosage forms can take the form of solutions, suspensions, emulsion, powders and the like. Such compositions and dosage forms will contain a prophylactically or therapeutically effective amount of a prophylactic or therapeutic agent, in certain embodiments, in purified form, together with a suitable amount of carrier so as to provide the form for proper administration to the subject. The formulation should suit the mode of administration. In one embodiment, the pharmaceutical compositions or single unit dosage forms are sterile and in suitable form for administration to a human or other subject.

A pharmaceutical composition provided herein is formulated to be compatible with its intended route of administration. Examples of routes of administration include, but are not limited to, parenteral routes (i.e., other than through the digestive tract), e.g., intravenous, intradermal, subcutaneous, intramuscular, inhalation, intranasal, transdermal, topical, transmucosal, intra-tumoral, and intra-synovial administration. In a specific embodiment, the composition is formulated in accordance with routine procedures as a pharmaceutical composition adapted for intravenous, subcutaneous, intramuscular, intranasal or topical administration to human beings. In certain embodiments, a pharmaceutical composition is formulated in accordance with routine procedures for subcutaneous administration to human beings. In one embodiment, pharmaceutical compositions for intravenous administration are solutions in sterile isotonic aqueous buffer. Where necessary, the pharmaceutical composition may also include a solubilizing agent and a local anesthetic such as lignocaine to ease pain at the site of the injection.

Examples of dosage forms include, but are not limited to: liquid dosage forms suitable for parenteral administration to a subject; and sterile solids (*e.g.*, crystalline or amorphous solids) that can be reconstituted to provide liquid dosage forms suitable for parenteral administration to a subject. An exemplary solid form is a lyophilized solid.

The pharmaceutical composition, shape, and type of dosage forms provided herein will typically vary depending on their use. For example, a dosage form used in the initial treatment of disease may contain larger amounts of one or more of the active ingredients it comprises than a dosage form used in the maintenance treatment of the same infection. Similarly, a parenteral dosage form may contain smaller amounts of one or more of the active ingredients it comprises than an oral dosage form used to treat the same disease or disorder. These and other ways in which specific dosage forms encompassed herein will vary from one another will be readily apparent to those skilled in the art. *See, e.g., Remington's Pharmaceutical Sciences.*

Generally, the ingredients of pharmaceutical compositions provided herein are supplied either separately or mixed together in unit dosage form, for example, as a dry lyophilized powder or water free concentrate in a hermetically sealed container such as an ampoule or sachette indicating the quantity of active agent. Where the pharmaceutical composition is to be administered by infusion, it can be dispensed with an infusion bottle containing sterile pharmaceutical grade water or saline. Where the pharmaceutical composition is administered by injection, an ampoule of sterile water for injection or saline can be provided so that the ingredients may be mixed prior to administration. In one embodiment, dosage forms provided herein comprise sufficient SNS-595 to permit administration of doses of SNS-595 within the range of about 10-100 $mg/m^2$ per day, or per week, given as a single once-a-day dose or as divided doses throughout the day, optionally taken with food.

In certain embodiments, the pharmaceutical dosage forms provided herein comprise a primary container comprising SNS-595. In certain embodiments, the primary container is within an opaque secondary container. In one embodiment, the primary container is a glass vial, such as a clear glass vial and the secondary container is an opaque foil-lined pouch, including an opaque metal foil-lined pouch, such as an opaque aluminum foil-lined pouch. In one embodiment, the pharmaceutical dosage forms provided herein comprise a clear glass vial comprising SNS-595, wherein the clear glass vial is within an opaque aluminum foil-lined pouch. Further, exemplary pharmaceutical dosage forms include those described in WO 2008/016668, incorporated herein by reference in its entirety. In one embodiment, the dosage forms provided herein comprise about 1-2000, 1-1000, 1-500, 1-300, 1-100 or 1-50 mg of SNS-595. Particular dosage forms provided herein comprise about 10, 15, 18, 21, 24, 25, 30, 40, 48, 50, 60, 70, 72, 75, 80, 90, 100, 150, 200, 300 or 500 mg of SNS-595.

It is understood that the foregoing detailed description and accompanying examples are merely illustrative, and are not to be taken as limitations upon the scope of the subject matter. Various changes and modifications to the disclosed embodiments will be apparent to those skilled in the art. Such changes and modifications, including without limitation

those relating to the methods of use provided herein, may be made without departing from the spirit and scope thereof. Patents, patent publications, and other publications referenced herein are incorporated by reference.

## 7. EXAMPLES

Certain embodiments of the claimed subject matter are illustrated by the following non-limiting examples.

The following cells lines were used in the examples described herein. All cell lines were cultured in Dulbecco's modified Eagle's medium, with the addition of 9% fetal calf serum and penicillin-streptomycin (90 U/mL) (DMEM), at 37 °C and 5% $CO_2$ atmosphere.

The SPD8 cell line carries a spontaneously derived mutation in the *hprt* gene and was isolated as a 6-thioguanine (6TG) resistant clone from the V79 Chinese hamster fibroblast cell line. The mutation within the *hprt* gene is a tandem duplication of exon 7, intron 6, and the 3' portion of exon 6 that results in expression of a non-functional HPRT protein (Darè et al., Somat Cell Mol Genet, 1996, 22:201-210; and Helleday et al., J Mol Biol, 1998, 279(4):687-694). This duplication can be lost through HR that revert the *hprt* gene to wild-type, which can be selected for in HAsT (50 μM hypoxanthine, 10 μM L-azaserine, 5 μM thymidine) (Helleday et al. 1998). The DMEM was supplemented with 6TG (5 μg/mL) in order to kill cells that undergo spontaneous reversion. The addition of 6TG at this concentration affects neither the growth rate of these mutants nor the recombination assay procedure.

The U-2 OS (human osteosarcoma) cell line was obtained from ATCC (HTB-96).

The VC8 and VC8-B2 cell lines originate from V79 Chinese hamster ovarian fibroblast cells. VC8 has a mutation in the *brca2* gene and VC8-B2 is this cell line complemented with the human chromosome 13 (containing the *brca2* gene; Kraakman-van der Zwet et al., Mol Cell Biol, 2002, 22(2):669-679).

The following chemicals were used in the assays described herein.

Aphidicolin (Sigma) powder dissolved in DMSO to no greater than 0.2%.

Doxorubicin (Sigma) and camptothecin (Sigma) were dissolved in dimethylsulfoxide (DMSO). The treatment dose did not exceed 0.2% of DMSO. Aliquots were kept at -20°C.

SNS-595 solution (10 mg SNS-595 per mL of aqueous solution of 4.5% D-sorbitol adjusted to pH 2.5 with methanesulfonic acid.) was kept at room temperature.

Treatment dilutions were made just prior to treatment in DMEM.

Gamma irradiation was performed in a $Cs^{137}$ chamber (1.9 Gy/min).

### **Example** 1: **Growth inhibition assay**

The influence of BRCA2 on sensitivity to SNS-595 was evaluated by a proliferation assay in Chinese hamster cells mutant (VC8) and complemented for functional BRCA2 (VC8-B2). Activity of SNS-595 in these assays was compared with doxorubicin. The following protocol was used for the assay:

Day 1

1. 4000 cells/well were plated in a 96-well plate such that half of the plate contained VC8 cells, and other half contained VC8-B2 cells. The last column was left cell-free as background control.
2. The plate was incubated at 37 °C with 5% $CO_2$.

Day 2

1. The media was removed and 50 μL of fresh media was added into each well.
2. In the first column, the media was removed and 200 μL of either 0.5 μM doxorubicin or 1.5 μM SNS-595 was added.
3. 100 μL from the first column was transferred into second column and mixed. 100 μL from the second column was transferred into third column and mixed and so on until end of plate. One column was left untreated. The plate was incubated at 37 °C with 5% $CO_2$.
4. After 4 hr treatment, plate was washed twice with 100 μL PBS and 250 μL media was added. The plate was incubated at 37°C with 5% $CO_2$.

Day 7

1. After 120 hr incubation, the plate was washed with 100 μL PBS and 100 μL resaszurin (10 μg/mL in phenol red-free complete DMEM) was added.
2. The plate was incubated at 37 °C with 5% $CO_2$ for 1 hr and fluorescence was measured at Em 530 nm/Ex 590 nm.

Figures 1 and 2 illustrate growth inhibition in Chinese hamster cells mutant (VC8) and complemented (VC8-B2) for functional BRCA2 in the presence of doxorubicin and SNS-595, respectively. In cells mutant for BRCA2, an approximately 5-fold increase in sensitivity was identified for SNS-595 as compared to cells expressing functional BRCA2 ($IC_{50}$ 0.14 μM vs. 0.72 μM). Doxorubicin sensitivity was increased approximately 4-fold ($IC_{50}$ 0.05 μM vs. 0.19 μM).

**Example 2: Colony outgrowth assay**

The influence of BRCA2 on sensitivity to SNS-595 was evaluated by colony outgrowth assay in U-2 OS cells, comparing wild-type cells to those depleted for BRCA2 using siRNA. Activity of SNS-595 in these assays was evaluated by clonogenic survival and compared with doxorubicin. The following protocol was used for the assay:

Day 1

1. 200,000 cells were plated in each of 2 wells in a 6-well plate, and incubated over night at 37 °C and 5% $CO_2$ atmosphere.

Day 2

1. The following reagents were prepared:

A: Test: 50 μL of 2 μM siBRCA2 (si Genome SMARTpool, Dharmacon) + 150 μL OptiMEM (Gibco)
B: Control (no siRNA): 200 μL OptiMEM
C: 2 μL DharmaFect 1 (Dharmacon) + 198 μL OptiMEM

2. Reagents were allowed to stand at room temperature for 5 minutes.
3. Reagents A+C and B+C were mixed and allowed to stand at room temperature for 20 min.
4. The media in wells was replaced with 1.6 mL of antibiotic-free media (DMEM, Gibco).
5. 400 μL of reagents A+B or B+C were added and mixed in wells. The plate was incubated at 37 °C.

Day 3

1. After 24 hr of siRNA treatment, cells were trypsinized and counted.
2. Cells were plated in 100 mm dishes at 500 or 1000 cells/dish in 10 mL media and incubated for 4 hr.
3. After 4 hr, test substances were added and plates were incubated for 14 days.

Day 17

1. The plates were harvested and cells were fixed in methylene blue (4g/L in methanol) and colonies containing more than 50 cells were counted.

The colony outgrowth in Figures 3 and 4 illustrates that U-2 OS cells depleted for BRCA2 using siRNA are 4.6X more sensitive to SNS-595 treatment than the wild-type cells.

**Example 3: Pulsed field gel electrophoresis (PFGE)**

Two million ($2 \times 10^6$) SPD8 cells were seeded in flasks (75 $cm^2$) and incubated overnight. Subsequently, cells were treated with SNS-595 (20 μM) or doxorubicin (3 μM) and in co-treatment with aphidicolin (3 μM) for 4 hr before being melted into agarose insert ($1 \times 10^6$ cells/70 μL 1% InCert Agarose, BMA). Inserts were transferred to 0.5 M EDTA, 1% N-laurylsarcosyl and proteinase K (1 mg/mL) and incubated at 50 °C for 48 hr and thereafter washed four times in TE-buffer (2 hr between each wash) prior to loading onto an agarose separation gel (1% Chromosomal grade agarose, Bio-Rad). Separation was performed on a CHEF DR III system (BioRad; 120° field angle, 240 seconds switch time, 4 V/cm) for 18 hr or for 24 hours with 60 to 240 seconds switch time. The gel was stained with ethidium bromide for 5 hr and subsequently analyzed by scanning fluorescence reader (Molecular Imager FX, BioRad) using Quantative One software.

Figure 5 and Figure 6 present data for PFGE analysis of cells treated with SNS-595, doxorubicin, and in co-treatment with aphidicolin. Figure 5 presents a PFGE run for 18 hr with 240 seconds switch time. Figure 6 presents a PFGE run for 24 hr with 60 to 240 seconds switch time. The data demonstrate production of more small DNA fragments in cells treated with doxorubicin as compared to those treated with SNS-595.

FIG. 7 presents data illustrating production of small DNA fragments following treatment with doxorubicin and SNS-595 for an 18 hr PFGE run with 240 seconds switch time. The difference in the DNA-damaging activity of SNS-595 and doxorubicin is demonstrated by production of more small DNA fragments in cells treated with doxorubicin as compared to those treated with SNS-595.

FIG. 8 presents data illustrating production of small DNA fragments following treatment with doxorubicin and SNS-595 for a 24 hr PFGE run with 60 to 240 seconds switch time. Again, cells treated with doxorubicin produce more small DNA fragments than those treated with SNS-595.

**Example 4: Recombination assay**

Each flask (75 $cm^2$) was inoculated with $1.5 \times 10^6$ SPD8 cells 24 hr prior to treatment with SNS-595 (2 μM) or doxorubicin (0.5 μM) and in co-treatment with aphidicolin (0.5 μM). After 4 hr of treatment, flasks were rinsed twice with PBS and 20 mL DMEM was added. Cells were then incubated for 48 hr to recover before plating onto Petri dishes. Cloning efficiency was measured by plating 500 cells in 10 mL of medium, two Petri dishes per dose. HPRT+ revertants were selected for by plating $3 \times 10^5$ cells/dish in the presence of HAsT (50 μM hypoxanthine, 10 μM L-azaserine, 5 μM thymidine), three dishes per dose. After 7 and 10 days, respectively, the plates were harvested and the colonies were fixed and stained using methylene blue in methanol (4 g/L). Colonies containing more than 50 cells were counted.

Figure 9 illustrates cloning efficiency of SPD8 cells upon treatment with aphidicolin, or with SNS-595 or doxorubicin, alone and in co-treatment with aphidicolin. As seen from the data, treatment with SNS-595 or doxorubicin significantly impaired cloning efficiency. Aphidicolin (S-phase blocker) caused a reduction in cytotoxicity for both drugs, this being more significant for SNS-595 (p=0.007) than doxorubicin (p=0.04). This demonstrates that a component of SNS-595 cytotoxicity is induced during S phase, and that the majority of cytotoxicity is S-phase independent.

Figure 10 illustrates reversion frequency of SPD8 cells upon treatment with aphidicolin, or with SNS-595 or doxorubicin, alone and in co-treatment with aphidicolin. Treatment with SNS-595 and doxorubicin increased the reversion frequency in SPD8 cells, which reflects the increased level of homologous recombination events. S-phase block (aphidicolin co-treatment) had no effect upon the reversion rate induced by SNS-595, but significantly reduced the doxorubicin-induced recombination events (p=0.04). This demonstrates that SNS-595-induced HRR recombination events are S-phase independent, in contrast with doxorubicin, in which a component of HRR-induced recombination is induced during S phase. These data further distinguish the molecular mechanism of action of SNS-595 from that of doxorubicin.

**Example 5: Pharmaceutical Composition Suitable for Injection or Intravenous Infusion**

An illustrative example of a suitable pharmaceutical composition comprises: 10 mg of SNS-595 and (+)-1,4-dihydro-7-[(3*S*,4*S*)-3-methoxy-4-amino-1-pyrrolidinyl]-4-oxo-1-(2-thiazolyl)-1,8-naphthyridine-3-carboxylic acid (wherein the amount of SNS-595 is at least 99.95% and the amount of (+)-1,4-dihydro-7-[(3*S*,4*S*)-3-methoxy-4-amino-1-pyrrolidinyl]-4-oxo-1-(2-thiazolyl)-1,8-naphthyridine-3-carboxylic acid is less than about 0.05%) per milliliter (mL) of an aqueous 4.5% solution of D-sorbitol, that is adjusted to pH 2.5 with methanesulfonic acid. One protocol for making such a solution includes the following for making a 100 mg/10 mL presentation: 100 mg of an active composition, which consists essentially of at least 99.95% SNS-595 and less than 0.05% (+)-1,4-dihydro-7-[(3*S*,4*S*)-3-methoxy-4-amino-1-pyrrolidinyl]-4-oxo-1-(2-thiazolyl)-1,8-naphthyridine-3-carboxylic acid, and 450 mg D-sorbitol are added to distilled water; the volume is brought up to a volume of 10 mL; and the resulting solution is adjusted to pH 2.5 with methanesulfonic acid. The resulting composition is also suitable for lyophilization. The lyophilized form is then reconstituted with sterile water to the appropriate concentration prior to use.

**Example 6: Pharmaceutical Composition Suitable for Injection or Intravenous Infusion**

An illustrative example of a suitable pharmaceutical composition comprises: 10 mg of total of SNS-595 and impurities (wherein the amount of SNS-595 is at least about 99.95% and the total amount of impurity is less than about 0.05%) per mL of aqueous solution of 4.5% sorbitol that is adjusted to pH 2.5 with methanesulfonic acid. One protocol for making such a solution includes the following for making a 100 mg/10 mL presentation: 100 mg composition consisting essentially of at least about 99.95% SNS-595 and less than about 0.05% impurities and 450 mg D-sorbitol are added to distilled water; the volume is brought up to a volume of 10 mL; and the pH of the resulting solution is adjusted to 2.5 with methanesulfonic acid. The resulting composition is also suitable for lyophilization. The lyophilized form is then reconstituted with sterile water to the appropriate concentration prior to use.

The embodiments of the claimed subject matter described above are intended to be merely exemplary, and those skilled in the art will recognize, or will be able to ascertain using no more than routine experimentation, numerous equivalents of specific compounds, materials, and procedures. All such equivalents are considered to be within the scope of the claimed subject matter and are encompassed by the appended claims.

The invention also relates to the following items:

1. A method for treating cancer in a subject having a BRCA2 mutation that impairs BRCA2 activity, comprising administering to the subject SNS-595 in a dose of about 10-100 mg/$m^2$.

2. The method of item 1, wherein the cancer is breast, ovarian, prostate, or pancreatic cancer.

3. The method of item 2, wherein the cancer is breast cancer.

4. The method of item 1, wherein the subject has a BRCA2 mutation selected from 999del5, 6174delT, 8803delC, 4486delG, 5445del5, 2024del5, 763insAT, 763insAT, 983delACAG, A3058T, 3758delACAG, 3908delTG, 4706delAAAG, 5804delTTAA, C6137A, 6174delT, 6305insA, 9132delC, del2352ins12, dup9700, and de11518.

5. The method of any of items 1-4, wherein SNS-595 is administered in a dose of about 40-90 mg/$m^2$.

6. The method of any of items 1-4, wherein SNS-595 is administered in a dose of about 30-60 mg/$m^2$.

7. The method of any of items 1-4, comprising i) administering a dose of about 40-90 mg/$m^2$ of SNS-595 to a subject; ii) waiting a period of at least six days where the subject is not administered any SNS-595; and iii) administering another dose of about 40-90 mg/$m^2$ of SNS-595 to the subject.

8. The method of any of items 1-4, wherein the dose is about 10 mg/$m^2$, about 15 mg/$m^2$, about 18 mg/$m^2$, about 21 mg/$m^2$, 24 mg/$m^2$, about 30 mg/$m^2$, about 35 mg/$m^2$, 40 mg/$m^2$, about 45 mg/$m^2$, about 48 mg/$m^2$, about 50 mg/$m^2$, about 60 mg/$m^2$, about 72 mg/$m^2$, about 75 mg/$m^2$, about 80 mg/$m^2$, about 85 mg/$m^2$, about 90 mg/$m^2$, or about 100 mg/$m^2$.

9. The method of any of items 1-8, wherein the dose of SNS-595 is administered once in three weeks.

10. The method of any of items 1-8, wherein the dose of SNS-595 is administered once in four weeks.

11. The method of any of items 1-8, wherein SNS-595 is administered in a dose of about 48 mg/$m^2$ once every three weeks.

12. The method of any of items 1-8, wherein SNS-595 is administered in a dose of about 60 mg/$m^2$ once every four weeks.

13. The method of any of items 1-5, wherein SNS-595 is administered in a dose of about 75 mg/$m^2$ once every four weeks.

14. The method of any of items 1-13, wherein SNS-595 is administered as an IV injection.

15. The method of any of items 1-14 further comprising administering a therapeutically effective dose of at least one other therapeutic agent.

16. The method of item 15, wherein the at least one other therapeutic agent is selected from alkylating agents, antimetabolites, aurora kinase inhibitors, purine antagonists, pyrimidine antagonists, spindle poisons, mitotic inhibitors, topoisomerase II inhibitors and poisons, topoisomerase I inhibitors, anti-neoplastic antibiotics, nitrosoureas, inorganic ion complexes, enzymes, hormones and hormone analogs, EGFR inhibitors, antibodies and antibody derivatives, IMIDs, HDAC inhibitors, Bcl-2 inhibitors, VEGF-stimulated tyrosine kinase inhibitors, VEGFR inhibitors, proteasome inhibitors, cyclin-dependent kinase inhibitors, PARP inhibitors, aromatase inhibitors, and dexamethasone.

17. The method of item 15, wherein the therapeutic agent is a support care agent.

18. A method of identifying a cancer subject suitable for treatment with SNS-595 comprising: (a) obtaining a biological sample from a candidate having cancer; (b) screening the biological sample for a BRCA2 mutation; and (c) if the candidate has a mutation that impairs activity of BRCA2, identifying the candidate as a cancer subject suitable for treatment with SNS-595.

19. The method of item 18 further comprising a step of treating the cancer subject with SNS-595.

20. A method of identifying one or more cancer subjects suitable for treatment with SNS-595 comprising: (a) obtaining biological samples from a plurality of candidates having cancer; (b) screening the biological samples for BRCA2 mutations; and (c) identifying each candidate having a BRCA2 mutation as a cancer subject suitable for treatment with SNS-595.

21. The method of any of items 18-20, wherein the biological sample comprises a body fluid sample or a tissue sample.

22. A method comprising contacting a cancer cell having a mutation that impairs BRCA2 activity with an amount of SNS-595 that is effective to induce double-strand DNA breaks in the cell.

23. A method for treating cancer in a subject having reduced BRCA2 activity, comprising administering to the subject SNS-595 in a dose of about 10-100 mg/$m^2$.

## Claims

**1.** (+)-1,4-Dihydro-7-[(3S,4S)-3-methoxy-4-(methylamino)-1-pyrrolidinyl]-4-oxo-1-(2-thiazolyl)-1,8-naphthyridine-3-carboxylic acid for use in a method for treatment of cancer in a subject having a BRCA2 mutation that impairs BRCA2 activity, wherein the method comprises administering to the subject (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid in a dose of about 10-100 mg/$m^2$.

**2.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to claim 1, wherein the cancer is breast, ovarian, prostate, or pancreatic cancer.

**3.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to claim 1, wherein the subject has a BRCA2 mutation selected from 999del5, 6174delT, 8803delC, 4486delG, 5445del5, 2024del5, 763insAT, 983delACAG, A3058T, 3758delACAG, 3908delTG, 4706delAAAG, 5804delTTAA, C6137A, 6174delT, 6305insA, 9132delC, de12352ins12, dup9700, and del1518.

**4.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to any of claims 1-3, wherein the method comprises administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid in a dose of about 40-90 mg/$m^2$, about 30-60 mg/$m^2$, or about 10 mg/$m^2$, about 15 mg/$m^2$, about 18 mg/$m^2$, about 21 mg/$m^2$, 24 mg/$m^2$, about 30 mg/$m^2$, about 35 mg/$m^2$, 40 mg/$m^2$, about 45 mg/$m^2$, about 48 mg/$m^2$, about 50 mg/$m^2$, about 60 mg/$m^2$, about 72 mg/$m^2$, about 75 mg/$m^2$, about 80 mg/$m^2$, about 85 mg/$m^2$, about 90 mg/$m^2$, or about 100 mg/$m^2$.

**5.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to any of claims 1-3, wherein the method comprises i) administering a dose of about 40-90 mg/$m^2$ of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid to a subject; ii) waiting a period of at least six days where the subject is not

administered any (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid; and iii) administering another dose of about 40-90 mg/$m^2$ of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid to the subject.

**6.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to any of claims 1-5, wherein the method comprises administration of the dose of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid once in three weeks, or once in four weeks.

**7.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to any of claims 1-5, wherein the method comprises administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid in a dose of about 48 mg/$m^2$ once every three weeks, about 60 mg/$m^2$ once every four weeks, or about 75 mg/$m^2$ once every four weeks.

**8.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to any of claims 1-7, wherein the method comprises administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid as an IV injection.

**9.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to any of claims 1-8, wherein the method further comprises administering a therapeutically effective dose of at least one other therapeutic agent.

**10.** The (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid for use according to claim 9, wherein the at least one other therapeutic agent is selected from alkylating agents, antimetabolites, aurora kinase inhibitors, purine antagonists, pyrimidine antagonists, spindle poisons, mitotic inhibitors, topoisomerase II inhibitors and poisons, topoisomerase I inhibitors, anti-neoplastic antibiotics, nitrosoureas, inorganic ion complexes, enzymes, hormones and hormone analogs, EGFR inhibitors, antibodies and antibody derivatives, IMIDs, HDAC inhibitors, Bcl-2 inhibitors, VEGF-stimulated tyrosine kinase inhibitors, VEGFR inhibitors, proteasome inhibitors, cyclin- dependent kinase inhibitors, PARP inhibitors, aromatase inhibitors, and dexamethasone, or wherein the therapeutic agent is a support care agent.

**11.** A method of identifying a cancer subject suitable for treatment with (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid comprising: (a) screening a biological sample for a BRCA2 mutation; and (b) if the candidate has a mutation that impairs activity of BRCA2, identifying the candidate as a cancer subject suitable for treatment with (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid.

**12.** A method of identifying one or more cancer subjects suitable for treatment with (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid comprising: (a) screening biological samples for BRCA2 mutations; and (b) identifying each candidate having a BRCA2 mutation as a cancer subject suitable for treatment with (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid.

**13.** The method of claim 11 or 12, wherein the biological sample comprises a body fluid sample or a tissue sample.

**14.** An in vitro method comprising contacting a cancer cell having a mutation that impairs BRCA2 activity with an amount of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid that is effective to induce double-strand DNA breaks in the cell.

**15.** (+)-1,4-Dihydro-7-[(3S,4S)-3-methoxy-4-(methylamino)-1-pyrrolidinyl]-4-oxo-1-(2-thiazolyl)-1,8-naphthyridine-3-carboxylic acid for use in a method for treatment of cancer in a subject having reduced BRCA2 activity, wherein the method comprises administering to the subject (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid in a dose of about 10-100 mg/$m^2$.

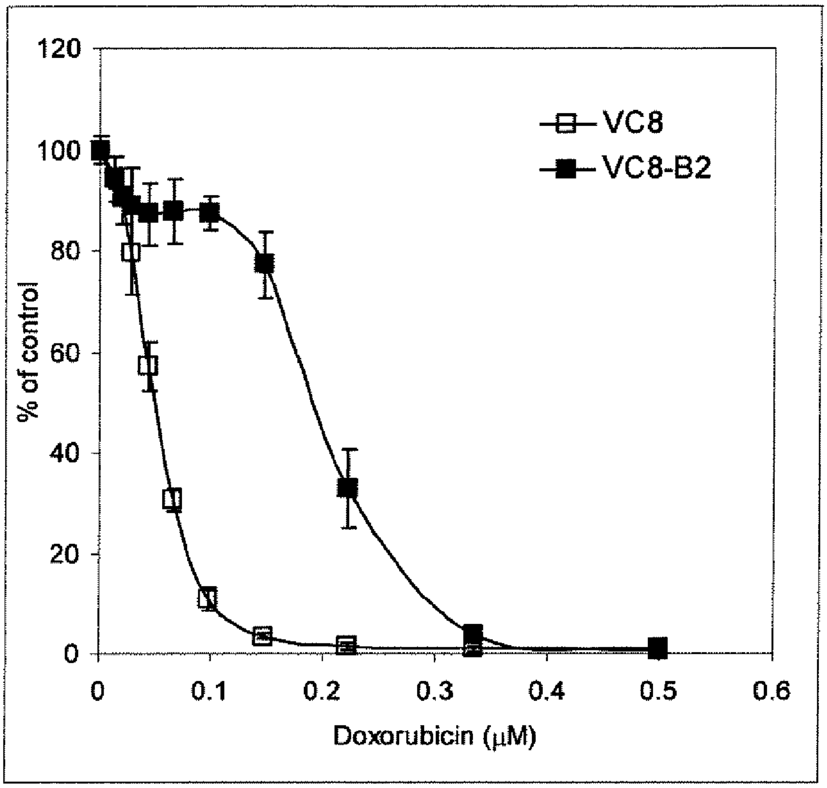
120
100
80
60
40
20
0
% of control
VC8
VC8-B2
0
0.1
0.2
0.3
0.4
0.5
0.6
Doxorubicin (μM)

FIGURE 2

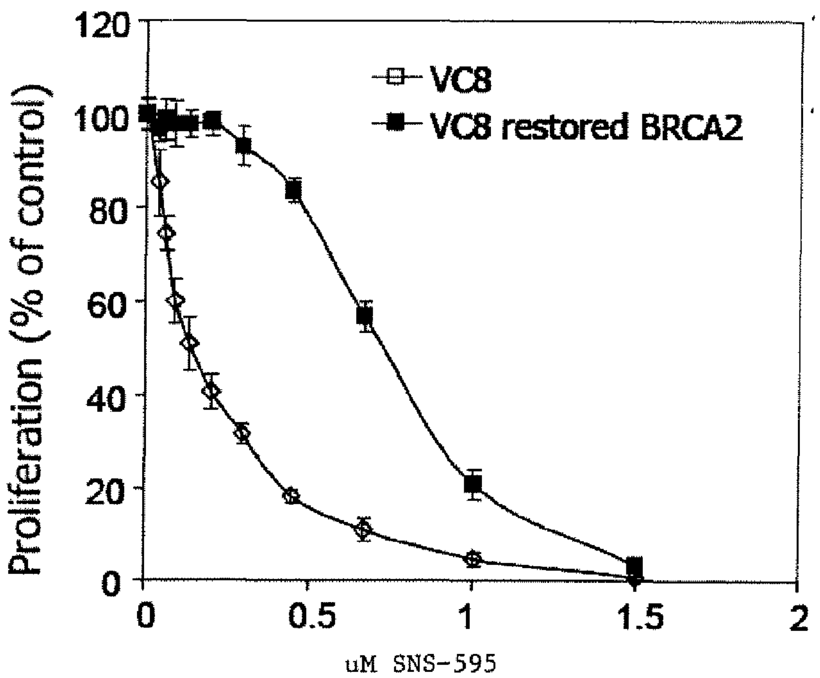
120
100
80
60
40
20
0
Proliferation (% of control)
VC8
VC8 restored BRCA2
0
0.5
1
1.5
2
uM SNS-595

FIGURE 3

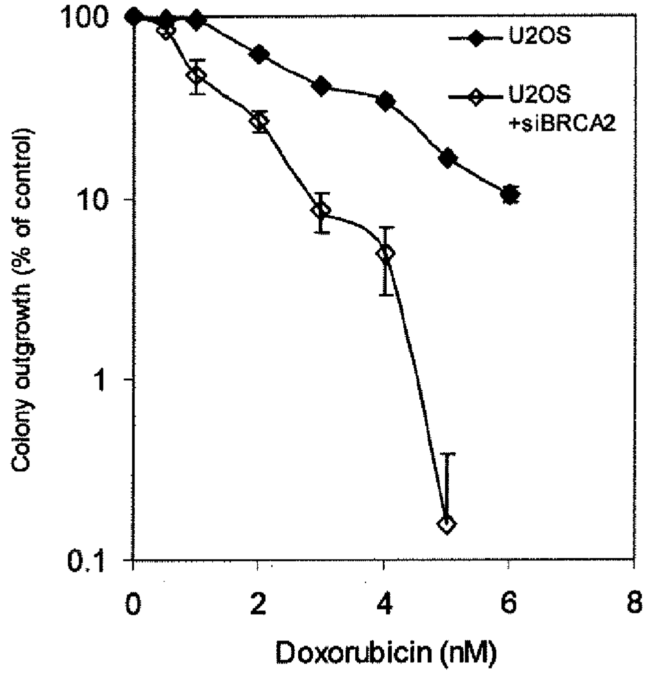
100
10
1
0.1
0
2
4
6
8
Colony outgrowth (% of control)
Doxorubicin (nM)
U2OS
U2OS
+siBRCA2

FIGURE 4

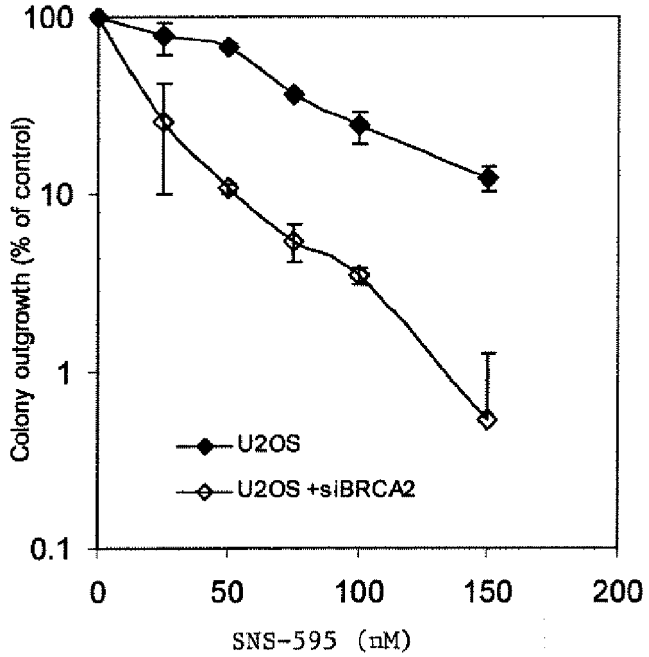
100
10
1
0.1
Colony outgrowth (% of control)
U2OS
U2OS +siBRCA2
0
50
100
150
200
SNS-595 (nM)

FIGURE 5

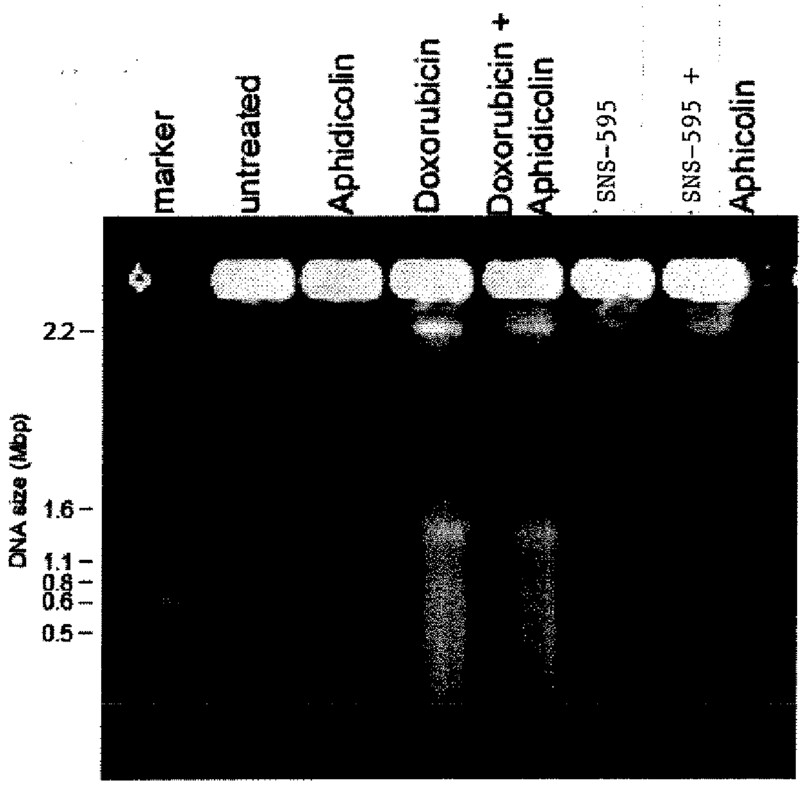
marker
untreated
Aphidicolin
Doxorubicin
Doxorubicin + Aphidicolin
SNS-595
SNS-595 + Aphicolin
DNA size (Mbp)
2.2
1.6
1.1
0.8
0.6
0.5

FIGURE 6

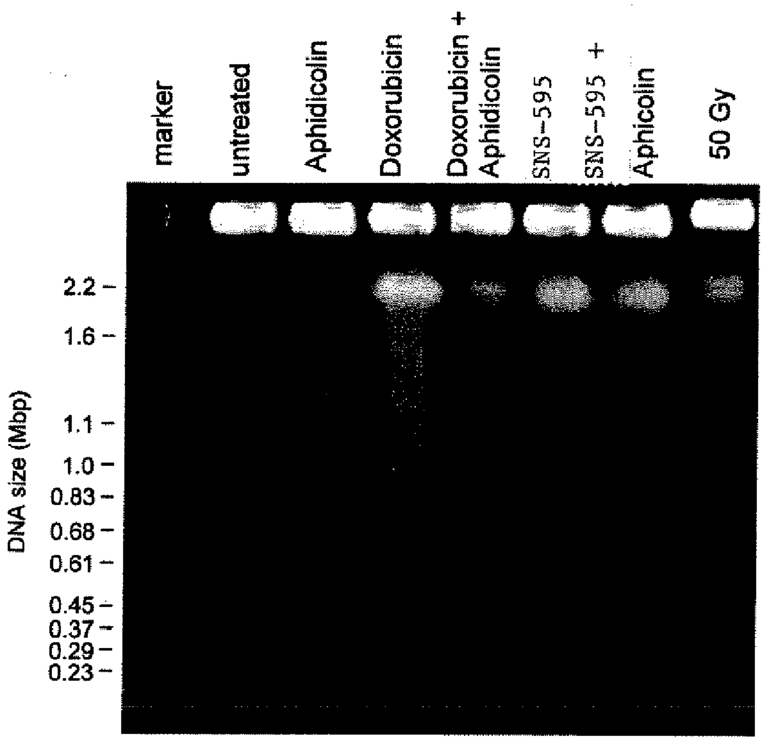
marker
untreated
Aphidicolin
Doxorubicin
Doxorubicin + Aphidicolin
SNS-595
SNS-595 + Aphicolin
50 Gy
DNA size (Mbp)
2.2
1.6
1.1
1.0
0.83
0.68
0.61
0.45
0.37
0.29
0.23

FIGURE 7

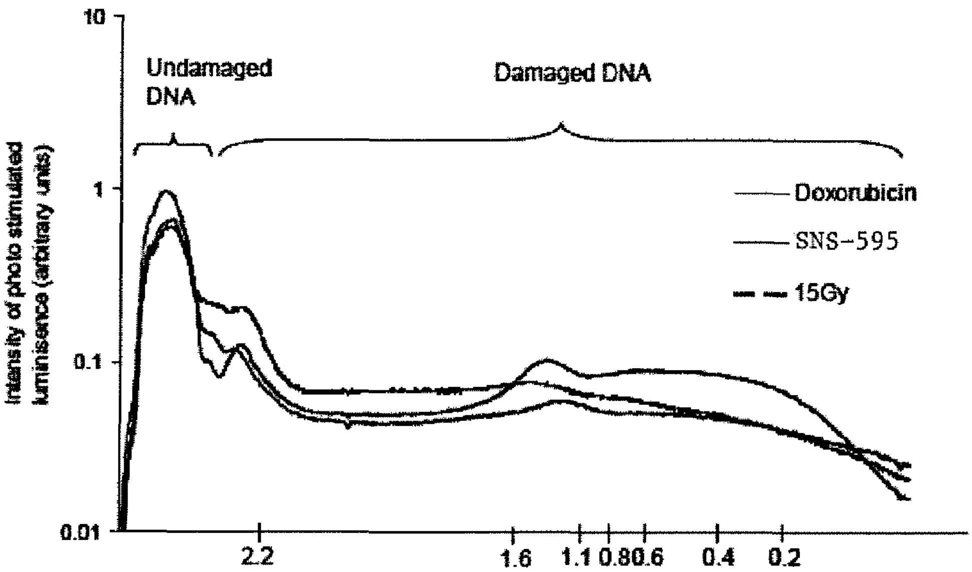
10
1
0.1
0.01
Intensity of photo stimulated luminisence (arbitrary units)
Undamaged DNA
Damaged DNA
Doxorubicin
SNS-595
15Gy
2.2
1.6
1.1
0.8
0.6
0.4
0.2

FIGURE 8

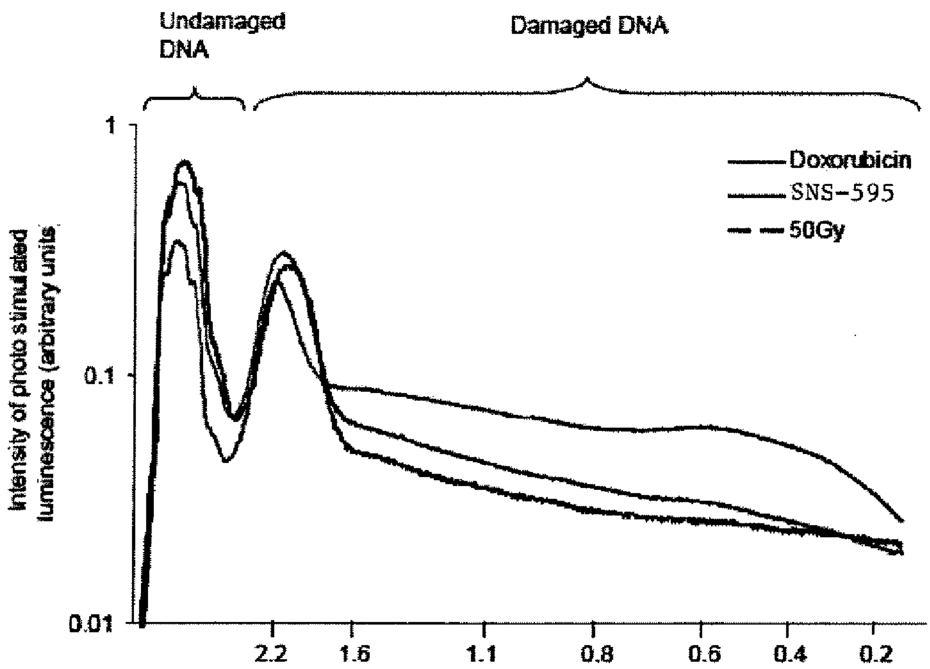
Undamaged DNA
Damaged DNA
Intensity of photo stimulated luminescence (arbitrary units)
1
0.1
0.01
2.2
1.6
1.1
0.8
0.6
0.4
0.2
Doxorubicin
SNS-595
50Gy

FIGURE 9

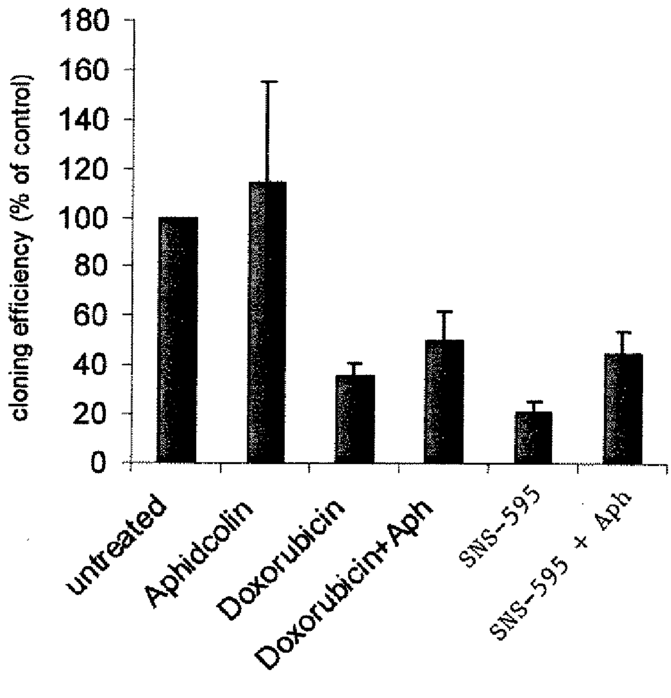
cloning efficiency (% of control)
180
160
140
120
100
80
60
40
20
0
untreated
Aphidcolin
Doxorubicin
Doxorubicin+Aph
SNS-595
SNS-595 + Aph

FIGURE 10

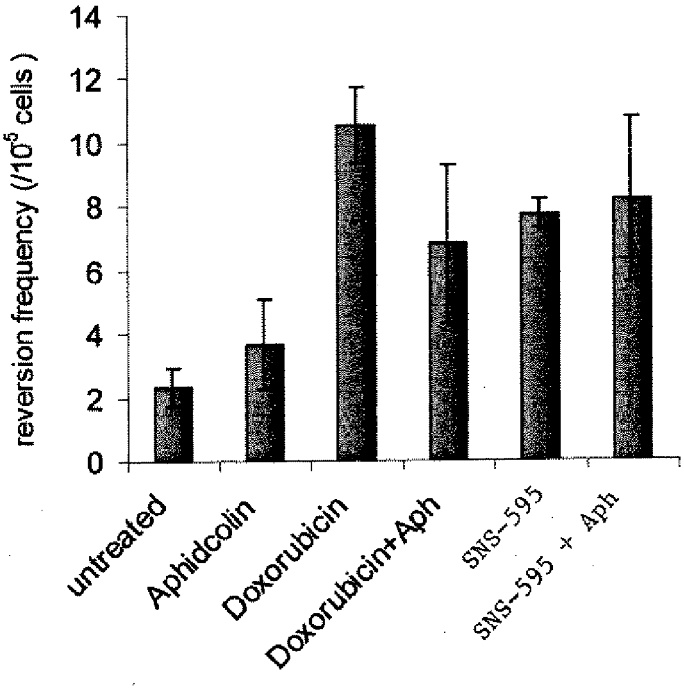
14
12
10
8
6
4
2
0
reversion frequency (/10^5 cells)
untreated
Aphidcolin
Doxorubicin
Doxorubicin+Aph
SNS-595
SNS-595 + Aph

Europäisches Patentamt
European Patent Office
Office européen des brevets

# EUROPEAN SEARCH REPORT

Application Number
EP 17 17 9348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MILLS DAVID A ET AL: "SNS-595, a naphthyridine cell cycle inhibitor and stimulator of apoptosis for the treatment of cancers", CURRENT OPINION IN INVESTIGATIONAL DRUGS, PHARMAPRESS, US, vol. 9, no. 6, 1 January 2008 (2008-01-01), pages 647-657, XP009118162, ISSN: 1472-4472 * page 647, column 1, paragraph 1 * * page 650, column 2, paragraph 2 * * page 651, column 2 - page 652, column 1, paragraph 1 * * page 655; table 2 * ----- | 1-15 | INV. A61K31/045 A61K31/4375 A61K45/06 A61P35/00 |
| A | US 2005/203120 A1 (ADELMAN DANIEL C [US] ET AL) 15 September 2005 (2005-09-15) * paragraph [0058]; example 2; table 1 * * claims 1-6,12-15,19, 20, 22 * ----- | 1-15 | |
| A | WO 2007/028171 A1 (SUNESIS PHARMACEUTICALS INC [US]; ADELMAN DANIEL C [US]; SILVERMAN JEF) 8 March 2007 (2007-03-08) * paragraph [0062] * * claims 33,34,36,39,41,43,44,47-49 * ----- | 1-15 | TECHNICAL FIELDS SEARCHED (IPC) A61K |
| A | POWELL S N ET AL: "Therapeutic exploitation of tumor cell defects in homologous recombination", ANTI-CANCER AGENTS IN MEDICINAL CHEMISTRY, BENTHAM SCIENCE PUBLISHERS LTD, NL, vol. 8, no. 4, 1 May 2008 (2008-05-01), pages 448-460, XP009134491, ISSN: 1871-5206 * page 456, column 2, paragraph 1 - page 457, column 1, paragraph 1 * ----- -/-- | 1-15 | |

The present search report has been drawn up for all claims

1

| Place of search | Date of completion of the search | Examiner |
|---|---|---|
| Munich | 20 November 2017 | Uryga-Polowy, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 17 9348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | HAWTIN RACHAEL E ET AL: "Voreloxin is active in breast cancer biopsies and potency is enhanced in a BRCA2 mutant background", PROCEEDINGS OF THE AMERICAN ASSOCIATION FOR CANCER RESEARCH ANNUAL MEETING, vol. 50, April 2009 (2009-04), page 411, XP002586408, & 100TH ANNUAL MEETING OF THE AMERICAN-ASSOCIATION-FOR-CANCER-RESEARCH; DENVER, CA, USA; APRIL 18 -22, 2009 ISSN: 0197-016X * the whole document * ----- | 1-15 | |

TECHNICAL FIELDS SEARCHED (IPC)

The present search report has been drawn up for all claims

1

| Place of search | Date of completion of the search | Examiner |
|---|---|---|
| Munich | 20 November 2017 | Uryga-Polowy, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 17 17 9348

This annex lists the patent family members relating to the patent documents cited in the above-mentioned European search report. The members are as contained in the European Patent Office EDP file on
The European Patent Office is in no way liable for these particulars which are merely given for the purpose of information.

20-11-2017

| Patent document cited in search report | Publication date | Patent family member(s) | Publication date |
|---|---|---|---|
| US 2005203120 A1 | 15-09-2005 | AT 451924 T | 15-01-2010 |
| | | AT 493982 T | 15-01-2011 |
| | | AU 2005222607 A1 | 29-09-2005 |
| | | AU 2005222622 A1 | 29-09-2005 |
| | | AU 2011201277 A1 | 07-04-2011 |
| | | BR PI0508700 A | 07-08-2007 |
| | | CA 2559650 A1 | 29-09-2005 |
| | | CA 2559652 A1 | 29-09-2005 |
| | | CA 2954578 A1 | 29-09-2005 |
| | | CN 1997368 A | 11-07-2007 |
| | | CN 101703508 A | 12-05-2010 |
| | | CN 103083316 A | 08-05-2013 |
| | | CN 103251589 A | 21-08-2013 |
| | | CN 104324029 A | 04-02-2015 |
| | | CY 1109741 T1 | 10-09-2014 |
| | | CY 1111263 T1 | 05-08-2015 |
| | | DK 1725233 T3 | 14-03-2011 |
| | | DK 1729770 T3 | 12-04-2010 |
| | | DK 2295056 T3 | 11-04-2016 |
| | | EP 1725233 A1 | 29-11-2006 |
| | | EP 1729770 A1 | 13-12-2006 |
| | | EP 2295056 A1 | 16-03-2011 |
| | | EP 3053579 A1 | 10-08-2016 |
| | | ES 2334802 T3 | 16-03-2010 |
| | | ES 2358267 T3 | 09-05-2011 |
| | | ES 2566973 T3 | 18-04-2016 |
| | | HK 1097196 A1 | 16-09-2011 |
| | | HU E027266 T2 | 28-10-2016 |
| | | IL 177946 A | 31-10-2016 |
| | | JP 5096913 B2 | 12-12-2012 |
| | | JP 5317472 B2 | 16-10-2013 |
| | | JP 5856644 B2 | 10-02-2016 |
| | | JP 2007529524 A | 25-10-2007 |
| | | JP 2007529528 A | 25-10-2007 |
| | | JP 2011225590 A | 10-11-2011 |
| | | JP 2012140442 A | 26-07-2012 |
| | | JP 2014148535 A | 21-08-2014 |
| | | JP 2015017110 A | 29-01-2015 |
| | | JP 2015227351 A | 17-12-2015 |
| | | JP 2017095471 A | 01-06-2017 |
| | | KR 20070033966 A | 27-03-2007 |
| | | NO 338058 B1 | 25-07-2016 |
| | | NZ 549557 A | 29-10-2010 |
| | | NZ 588060 A | 30-09-2011 |
| | | PT 1725233 E | 23-03-2011 |
| | | PT 1729770 E | 08-02-2010 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 17 17 9348

This annex lists the patent family members relating to the patent documents cited in the above-mentioned European search report. The members are as contained in the European Patent Office EDP file on
The European Patent Office is in no way liable for these particulars which are merely given for the purpose of information.

20-11-2017

| Patent document cited in search report | Publication date | Patent family member(s) | Publication date |
|---|---|---|---|
| | | SI 1725233 T1 | 29-04-2011 |
| | | SI 1729770 T1 | 26-02-2010 |
| | | US 2005203120 A1 | 15-09-2005 |
| | | US 2005215583 A1 | 29-09-2005 |
| | | US 2006247267 A1 | 02-11-2006 |
| | | US 2011263524 A1 | 27-10-2011 |
| | | US 2011312988 A1 | 22-12-2011 |
| | | US 2013244967 A1 | 19-09-2013 |
| | | US 2014378505 A1 | 25-12-2014 |
| | | US 2016361302 A1 | 15-12-2016 |
| | | WO 2005089756 A1 | 29-09-2005 |
| | | WO 2005089757 A1 | 29-09-2005 |
| | | ZA 200607248 B | 25-02-2009 |
| WO 2007028171 A1 | 08-03-2007 | AU 2006287149 A1 | 08-03-2007 |
| | | EP 1931339 A1 | 18-06-2008 |
| | | WO 2007028171 A1 | 08-03-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61156449 A
- US 61170013 B
- US 61141856 B
- US 12650390 B
- WO 2007146335 A
- US 5817669 A
- JP 10173986 A
- US 20050203120 A
- US 20050215583 A
- US 20060025437 A
- US 20060063795 A
- US 20060247267 A
- US 61240161 B
- US 61240113 B
- US 61288213 B
- US 5134127 A
- WO 2008016668 A


### Non-patent literature cited in the description


- **LI ; KARLAN.** *Curr Oncol Rep,* 2001, vol. 3, 27-32
- **NATHANSON et al.** *Nature Med,* 2001, vol. 7, 552-556
- **VENKITARAMAN.** *J. Cell Sci,* 2001, vol. 114, 3591-98
- **THOMPSON et al.** *Mutation Research/Fundamental and Molecular Mechanisms of Mutagenesis,* 2001, vol. 477, 131-153
- **DUFRESNE et al.** *Arch Pathol Lab Med,* 2006, vol. 130, 185-187
- **TAKANO et al.** *BMC Cancer,* 2008, vol. 8, 59
- **LOMAN et al.** *J Natl Cancer Inst,* 2001, vol. 93 (16), 1215-1223
- **PETO et al.** *J Natl Cancer Inst,* 1999, vol. 91 (11), 943-949
- **WALSH et al.** *JAMA,* 2006, vol. 295 (12), 1379-88
- **GEWIRTZ D.** *Biochem Pharmacol,* 1999, vol. 57, 727-41
- **O'REILLY et al.** *Biochemistry,* 2002, vol. 41, 7989-97
- **WANG J.** *Nat Rev Mol Cell Biol,* 2002, vol. 3, 430-40
- *Oncogene,* 1998, vol. 16 (23), 3069-82
- **KUZNETSOV et al.** *Nature Medicine,* 2008, vol. 14, 875-881
- Remington's Pharmaceutical Sciences. Mack Publishing, 2005
- **DARÈ et al.** *Somat Cell Mol Genet,* 1996, vol. 22, 201-210
- **HELLEDAY et al.** *J Mol Biol,* 1998, vol. 279 (4), 687-694
- **KRAAKMAN-VAN DER ZWET et al.** *Mol Cell Biol,* 2002, vol. 22 (2), 669-679